# EUROPÄISCHE PATENTANMELDUNG

(11) **EP 1 770 087 A1**
(43) Veröffentlichungstag der Anmeldung: **04.04.2007**
(21) Anmeldenummer: 05021282.8
(22) Anmeldetag: 29.09.2005
(51) Int. Cl.: C07D 249/12, C07D 401/12, C07D 403/12, C07D 401/14, C07D 243/10

(54) **Ausgewählte CGRP-Antagonisten, Verfahren zu deren Herstellung sowie deren Verwendung als Arzneimittel**

(71) Anmelder: Boehringer Ingelheim Pharma GmbH & Co. KG, 55216 Ingelheim (DE)
(72) Erfinder: Die Erfindernennung liegt noch nicht vor

(57) **Zusammenfassung**

Gegenstand der vorliegenden Erfindung sind die CGRP-Antagonisten der allgemeinen Formel I 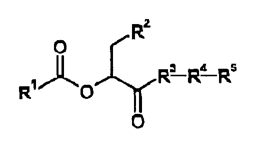 in der **R¹, R², R³, R⁴** und **R⁵** wie in Anspruch 1 definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, sowie diejenigen Verbindungen der allgemeinen Formel in denen ein oder mehrere Wasserstoffatome durch Deuterium ausgetauscht sind, diese Verbindungen enthaltende Arzneimittel, deren Verwendung und Verfahren zu ihrer Herstellung.

## Beschreibung

Gegenstand der vorliegenden Erfindung sind die CGRP-Aniaganisten der allgemeinen Formel **I** 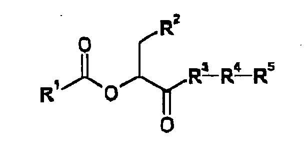 in der **R¹, R², R³, R⁴** und **R⁵** wie in Anspruch 1 definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, sowie diejenigen Verbindungen der allgemeinen Formel **I**, in denen ein oder mehrere Wasserstoffatome durch Deuterium ausgetauscht sind, diese Verbindungen enthaltende Arzneimittel, deren Verwendung und Verfahren zu ihrer Herstellung.

### STAND DER TECHNIK

In den internationalen Patentanmeldungen PCT/EP97/04862 und PCT/EP04/000087 werden bereits CGRP-Antagonisten zur Behandlung von Migräne beschrieben.

### DETAILLIERTE BESCHREIBUNG DER ERFINDUNG

In der obigen allgemeinen Formel **I** bedeuten in einer ersten Ausführungsform
- **R¹**: eine Gruppe ausgewählt aus 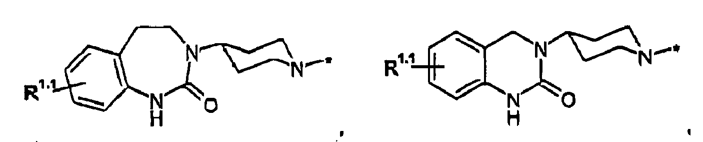 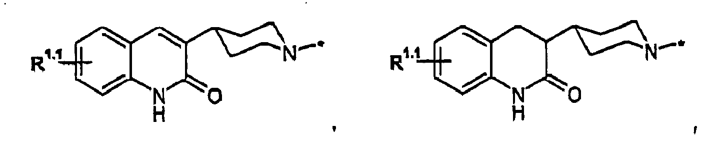 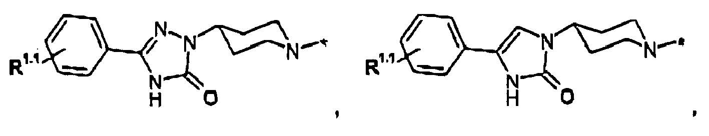 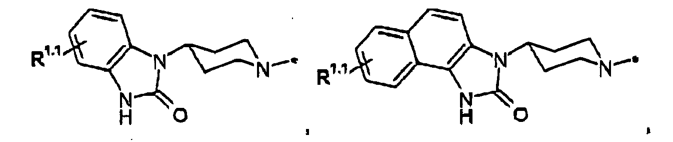 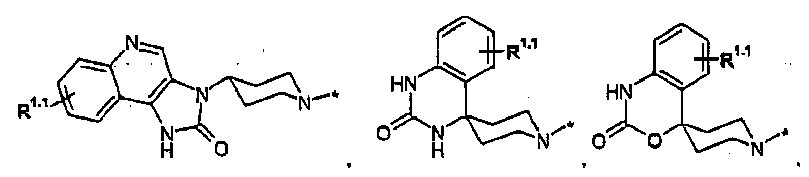 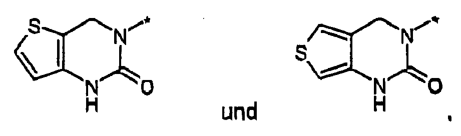 worin
**R^{1.1}** H, Halogen, HO-, F₃C- oder C₁₋₆-Alkyl-O- darstellt,
- **R²**: eine Gruppe der allgemeinen Formeln **II** 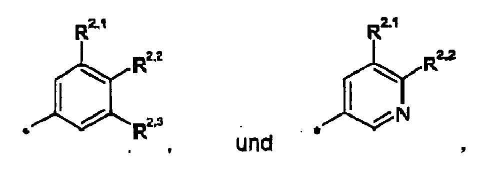 worin
R^{2.1} H, Halogen, C₁₋₃-Alkyl-O-, C₁₋₃-Alkyl- oder F₃C-,
R^{2.2} H, H₂N-, HO-, H₃C-O-, H-C(O)-O- oder C₁₋₃-Alkyl-C(O)-O-,
R^{2.3} H, Halogen, C₁₋₃-Alkyl- oder F₃C- darstellt,
- R³: eine Gruppe der allgemeinen Formeln **III** 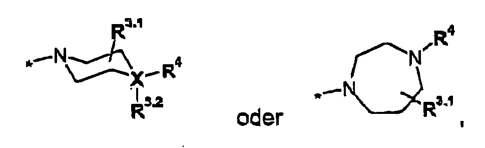 worin
X N oder C,
**R^{3.1}** H, C₁₋₃-Alkyl- oder **R^{3.1.1}**-(O)C-,
**R^{3.1.1}** HO- oder C₁₋₆-Alkyl-O-,
**R^{3.2}** ein freies Elektronenpaar , wenn **X** = **N** ist, oder
**R^{3.2}** H oder C₁₋₃-Alkyl- darstellt, wenn **X** = **C** ist,
- **R⁴**: eine Gruppe ausgewählt aus 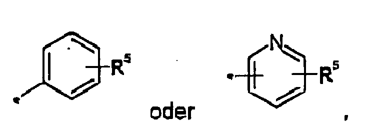
- **R⁵**: R^{5.1}-O-C(O)- und
**R^{5.1}** H, C₁₋₈-Alkyl-, Phenyl-, Indanyl-, Pyridyl-C₁₋₃-alkylenyl-, HO-C₂₋₄-alkylenyl-, C₁₋₈-Alkyl-O-C₂₋₄-alkylenyl-, HO-C₂₋₄-alkylenyl-O-C₂₋₄-alkylenyl-, C₁₋₆-Alkyl-C₂₋₄-alkylenyl-O-C₂₋₄-alkylenyl-, H₂N-C₂₋₄-alkylenyl--, (C₁₋₆-Alkyl)-NH-C₂₋₄-alkylenyl-, (C₁₋₆-Alkyl)₂N-C₂₋₄-alkylenyl-, H₂N-C(O)-C₁₋₃-alkylenyl-, (C₁₋₃-Alkyl)-NH-C(O)-C₁₋₃-alkylenyl-, (C₁₋₆-Alkyl)₂N-C(O)-C₁₋₃-alkylanyl-, C₁₋₆-Alkyl-C(O)-O-C₁₋₃-alkylenyl-, C₁₋₆-Alkyl-O-C(O)-O-C₁₋₃-alkylenyl-, **R^{5.1.1}**-C(O)-C₁₋₃-alkylenyl- oder **R^{5.1.2}**-C₂₋₄-alkylenyl-.
**R^{5.1.1}** eine Gruppe ausgewählt aus 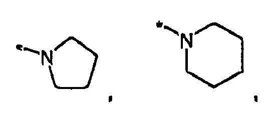 **R^{5.1.2}** eine Gruppe ausgewählt aus 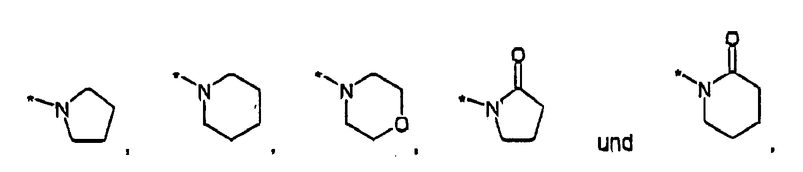
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zweite Ausführungsform der vorliegenden Erfindung besteht in den Verbindungen der obigen allgemeinen Formel **I**, in denen
- **R¹**: eine Gruppe ausgewählt aus 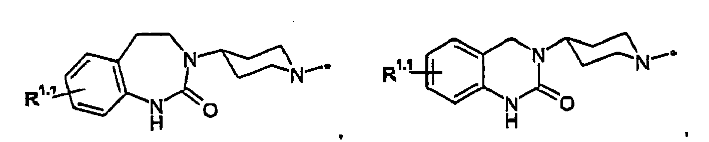 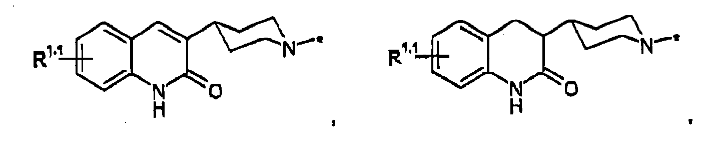 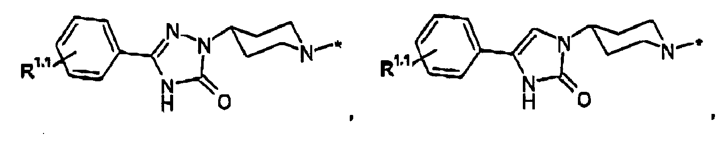 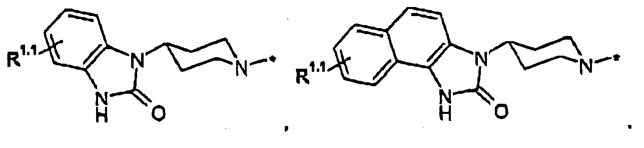 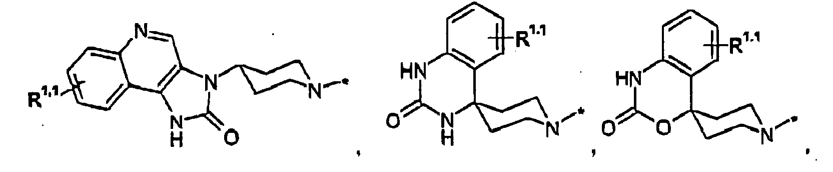 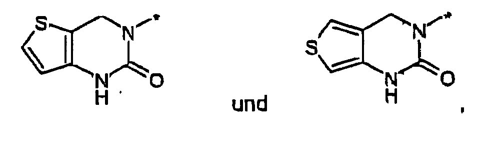 worin
**R^{1.1}** H oder H₃C-O- darstellt,
- **R²**: eine Gruppe ausgewählt aus 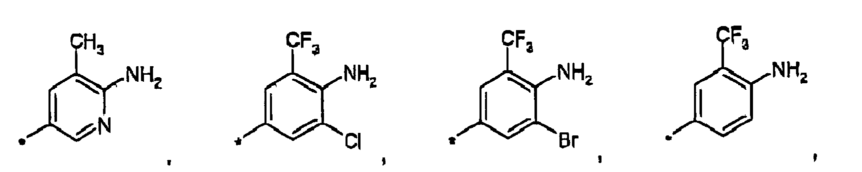 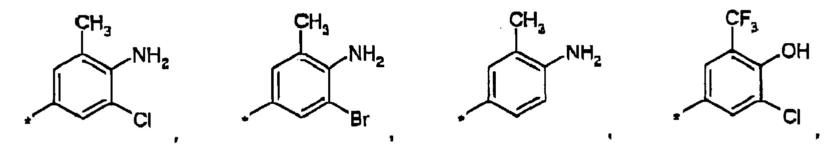 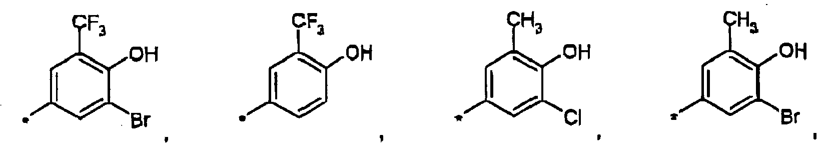 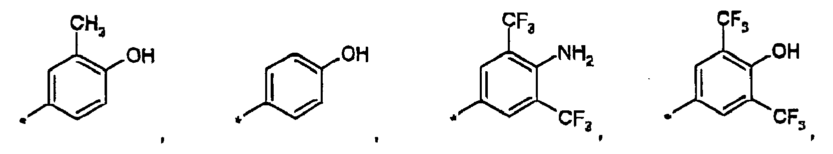 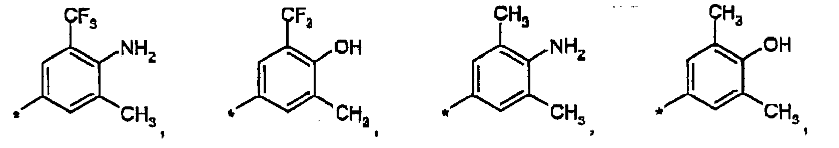 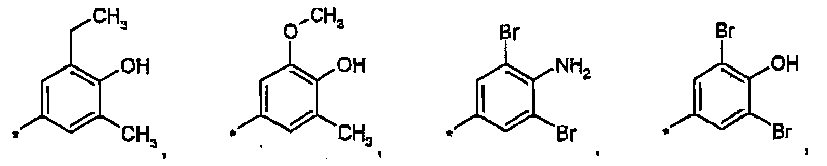 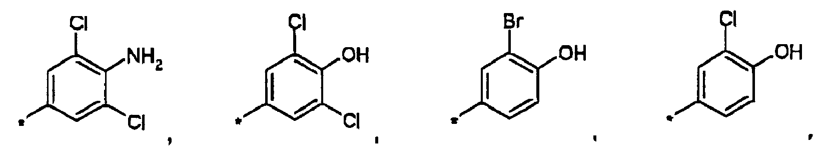 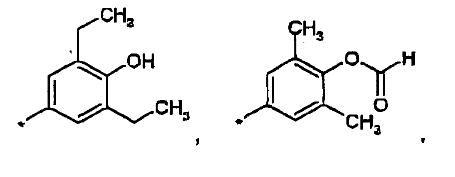 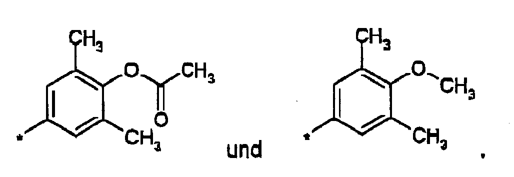
- **R³-R⁴**: zusammen eine Gruppe ausgewählt aus 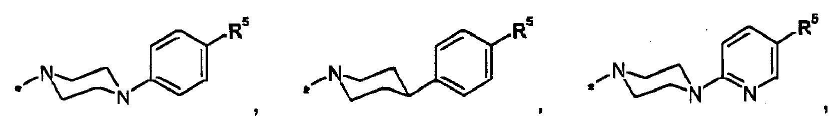 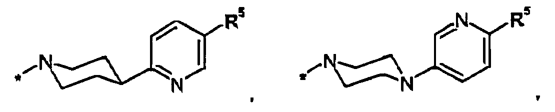 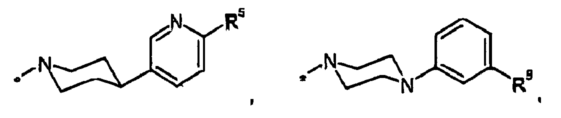 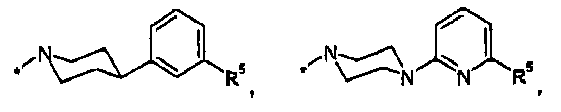 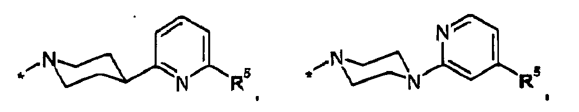 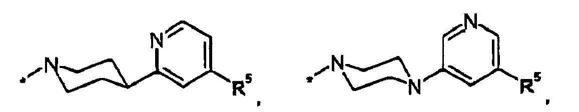 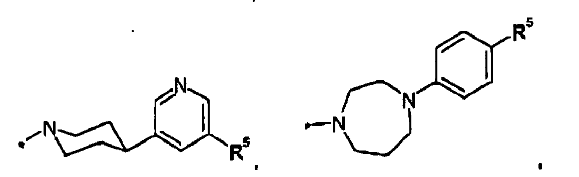 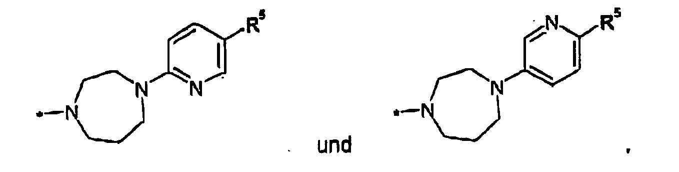
- **R⁵**: **R^{5.1}** -O-C(O)- und
**R^{5.1}** H, C₁₋₈-Alkyl-, Phenyl-, Indanyl-, Pyridyl-C₁₋₃-alkylenyl-, HO-C₂₋₄-alkylenyl-, C₁₋₆-Alkyl-O-C₂₋₄-alkylenyl-. HO-C₂₋₄-alkylenyl-O-C₂₋₄-alkylenyl-, C₁₋₈-Alkyl-C₂₋₄-alkylenyl-O-C₂₋₄-alkylenyl-. H₂N-C₂₋₄-alkylenyl-, (C₁₋₆-Alkyl)-NH-C₂₋₄-alkylenyl-, (C₁₋₆-Alkyl)₂N-C₂₋₄-alkylenyl-, H₂N-C(O)-C₁₋₃-alkylenyl-, (C₁₋₆-Alkyl)-NH-C(O)-C₁₋₃-alkylenyl-, (C₁₋₆-Alkyl)₂N-C(O)-C₁₋₃-alkylenyl-, G₁₋₆-Alkyl-C(O)-O-C₁₋₃alkylenyl-, C₁₋₆-Alkyl-O-C(O)-C₁₋₃-alkylenyl-, **R^{5.1.1}**-C(O)-C₁₋₃-alkylenyl- oder **R^{5.1.2}**-C₂₋₄-alkylenyl-.
**R^{5.1.1}** eine Gruppe ausgewählt aus 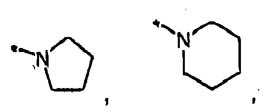 **R^{5.1.2}** eine Gruppe ausgewählt aus 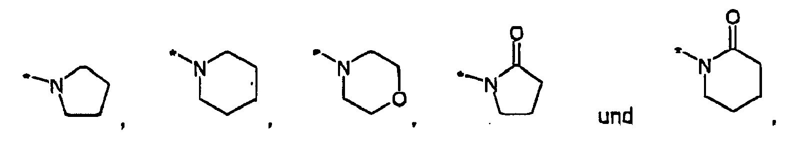
bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dritte Ausführungsform der vorliegenden Erfindung besteht in den Verbindungen der obigen allgemeinen Formel **I**, in denen
- **R¹**: eine Gruppe ausgewählt aus 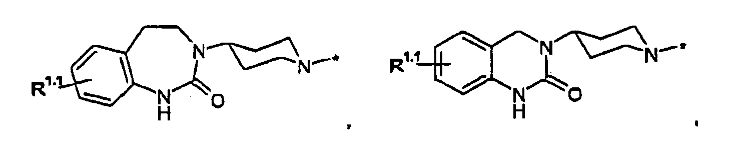 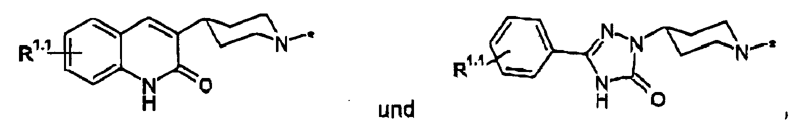
- **R²**: eine Gruppe ausgewählt aus 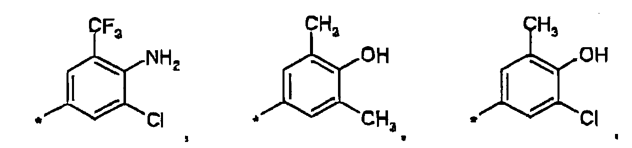 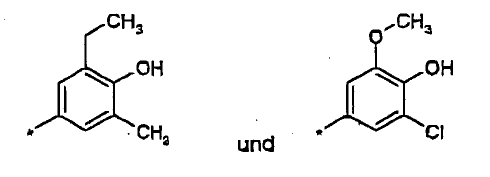
- **R³-R⁴**: zusammen eine Gruppe ausgewählt aus 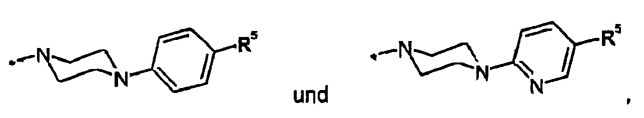
- **R⁵**: **R^{5.1}**-O-C(O)- und
**R^{5.1}** H, C₁₋₈-Alkyl-, Phenyl-, Indanyl-, Pyridyl-CH₂-, C₁₋₃-Alkyl-O-C₂₋₄-alkylenyl-, C₁₋₃-Alkyl-O-C₂₋₄-alkylenyl-O-C₂₋₄-alkylenyl-, (C₁₋₃-Alkyl)₂N-C₂₋₄-alkylenyl-, (C₁₋₃-Alkyl)₂N-C(O)-C₁₋₃-alkylenyl-, C₁₋₆-Alkyl-C(O)-O-C₁₋₃-alkylenyl-, C₁₋₃-Alkyl-O-C(O)-O-C₁₋₃-alkylenyl-, **R^{5.1.1}**-C(O)-C₁₋₃-alkylenyl- oder **R^{5.1.2}**-C₂₋₄-alkylenyl-,
**R^{5.1.1}** eine Gruppe ausgewählt aus 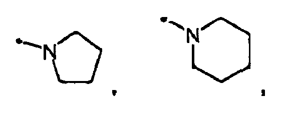 **R^{5.1.2}** eine Gruppe ausgewählt aus 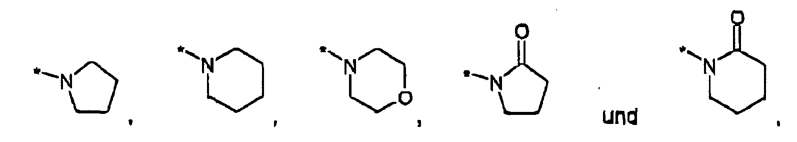
bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel **I** seien beispielsweise weiterhin folgende Verbindungen genannt:

| **Nr.** | **Struktur** |
|---|---|
| (1) | 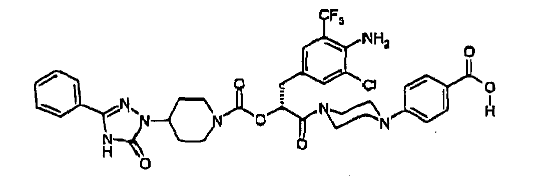 |
| (2) | 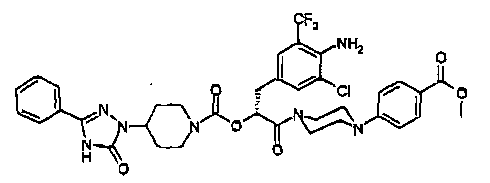 |
| (3) | 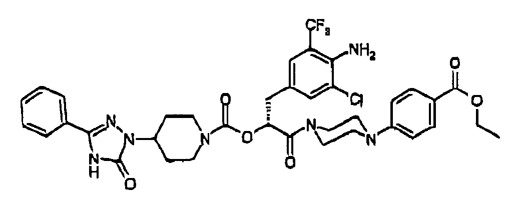 |
| (4) | 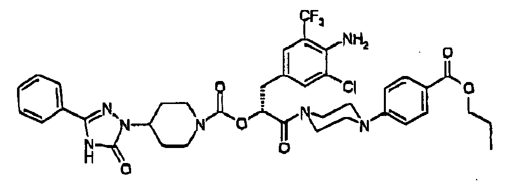 |
| (5) | 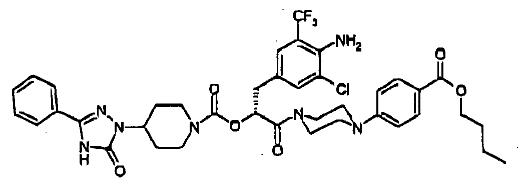 |
| (6) | 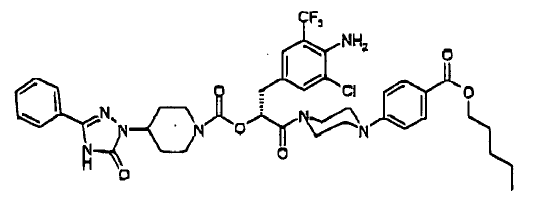 |
| (7) | 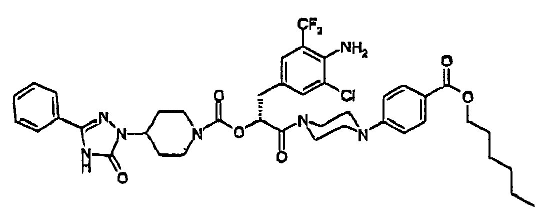 |
| (8) | 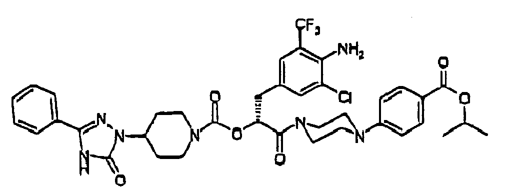 |
| (9) | 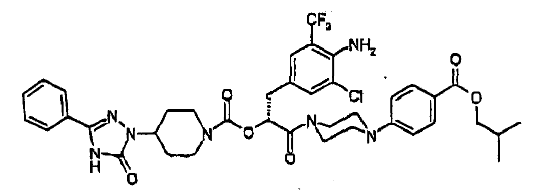 |
| (10) | 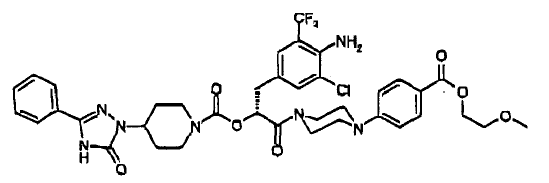 |
| (11) | 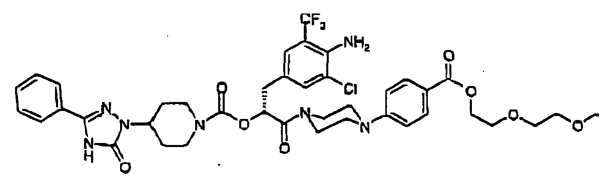 |
| (12) | 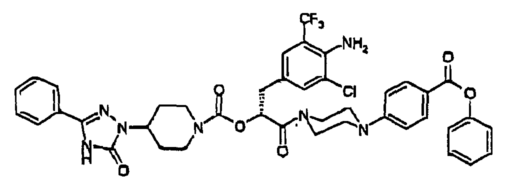 |
| (13) | 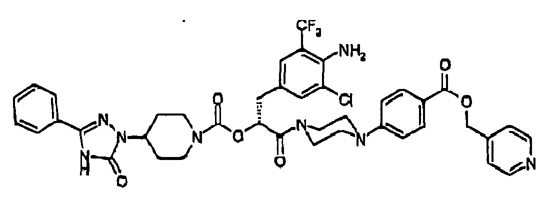 |
| (14) | 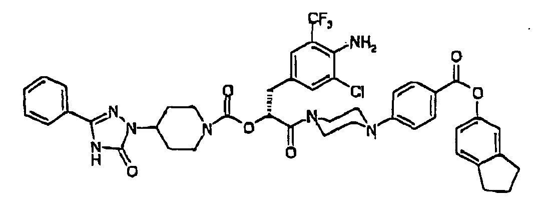 |
| (15) | 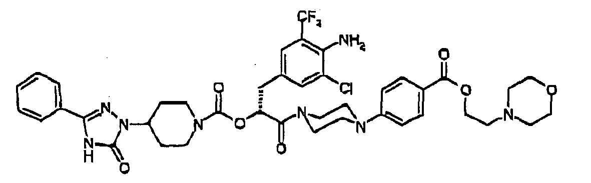 |
| (16) | 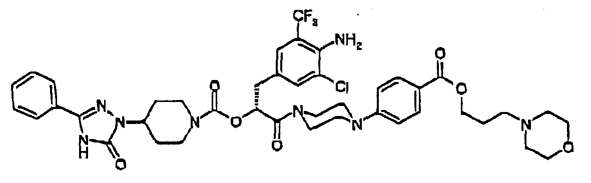 |
| (17) | 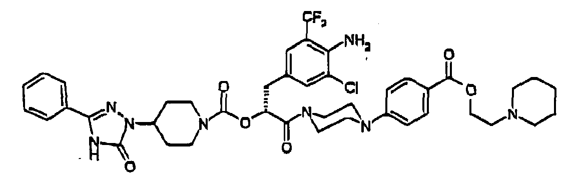 |
| (18) | 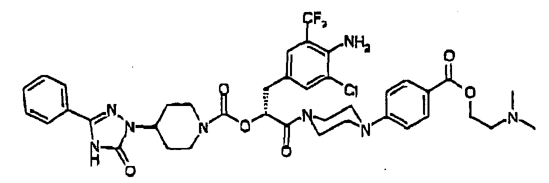 |
| (19) | 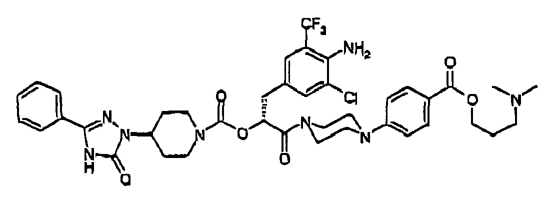 |
| (20) | 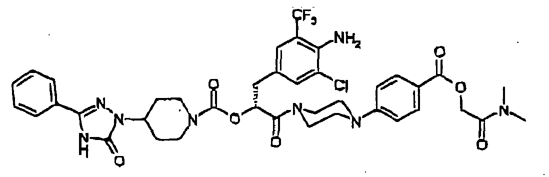 |
| (21) | 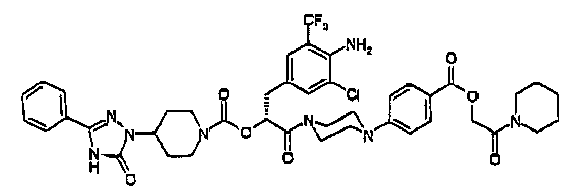 |
| (22) | 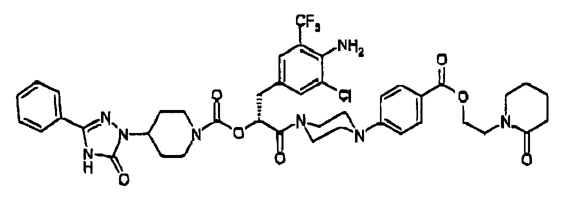 |
| (23) | 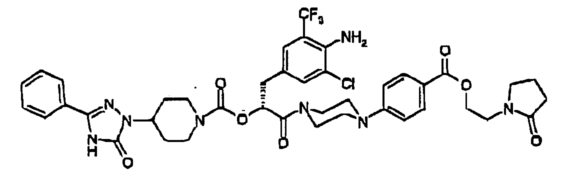 |
| (24) | 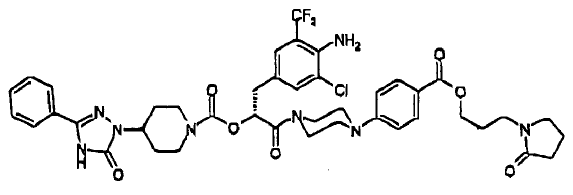 |
| (25) | 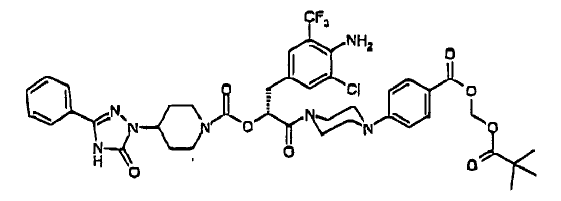 |
| (26) | 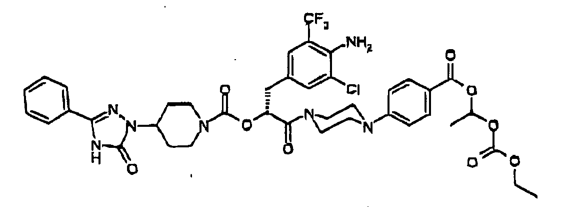 |
| (27) | 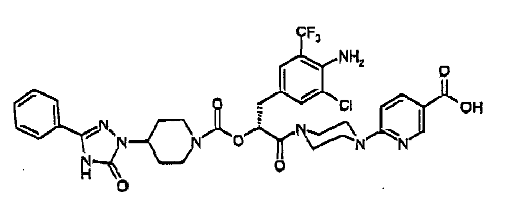 |
| (28) | 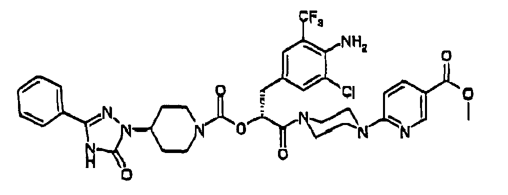 |
| (29) | 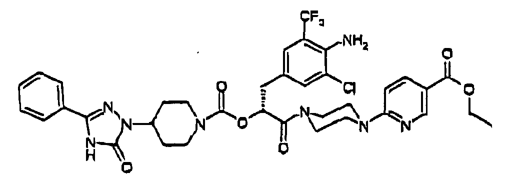 |
| (30) | 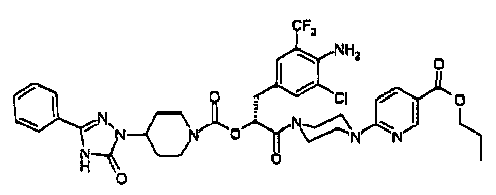 |
| (31) | 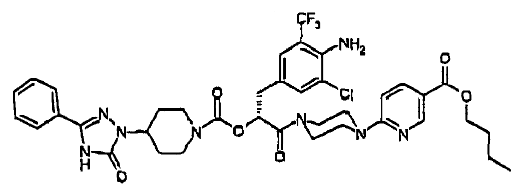 |
| (32) | 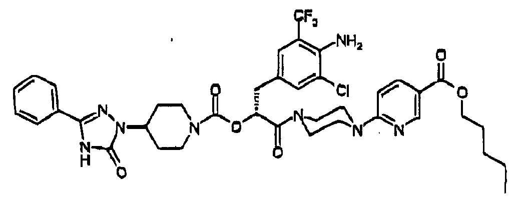 |
| (33) | 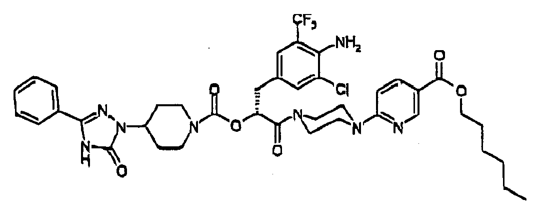 |
| (34) | 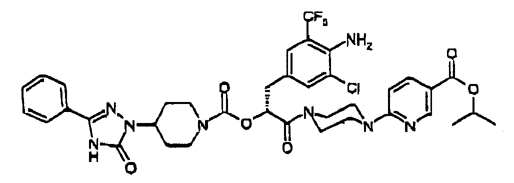 |
| (35) | 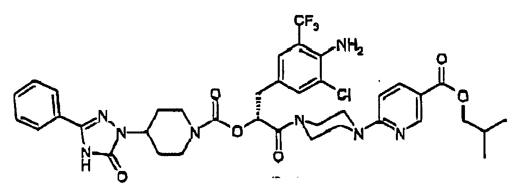 |
| (36) | 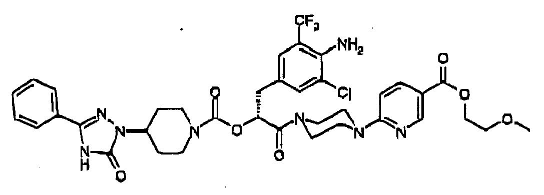 |
| (37) | 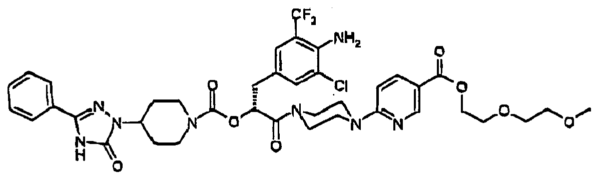 |
| (38) | 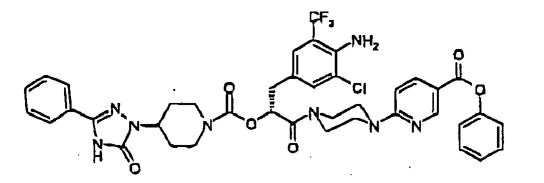 |
| (39) | 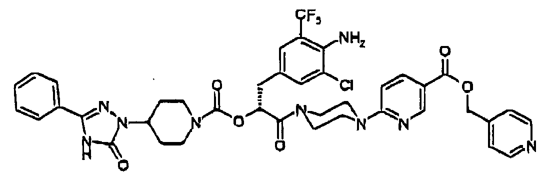 |
| (40) | 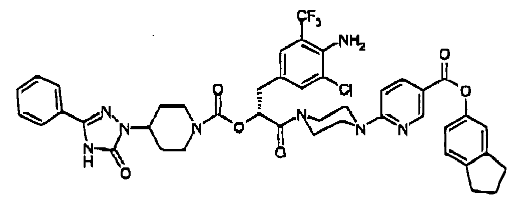 |
| (41) | 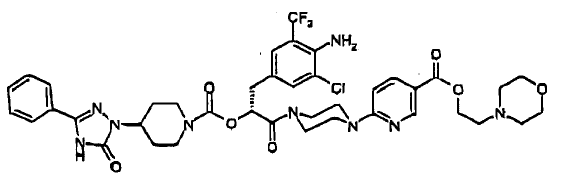 |
| (42) | 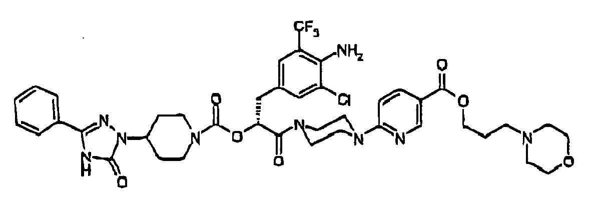 |
| (43) | 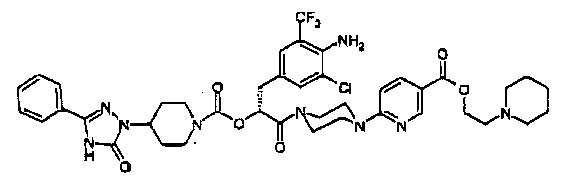 |
| (44) | 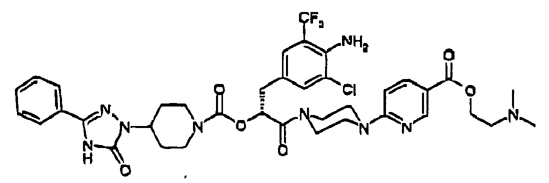 |
| (45) | 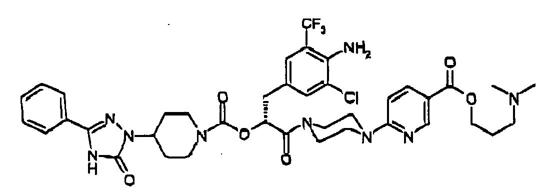 |
| (46) | 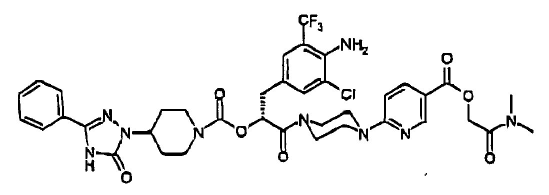 |
| (47) | 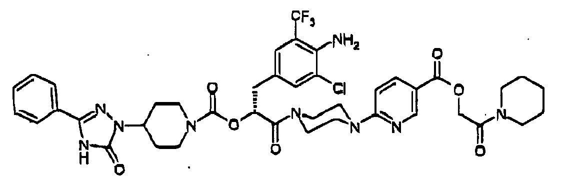 |
| (48) | 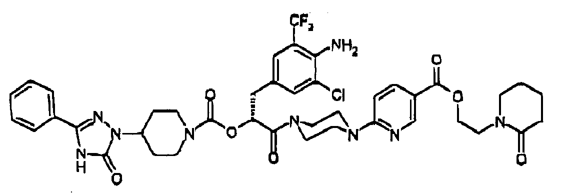 |
| (49) | 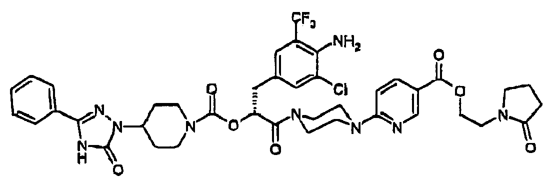 |
| (50) | 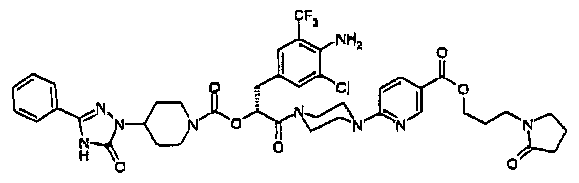 |
| (51) | 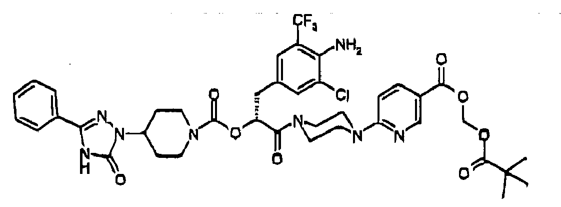 |
| (52) | 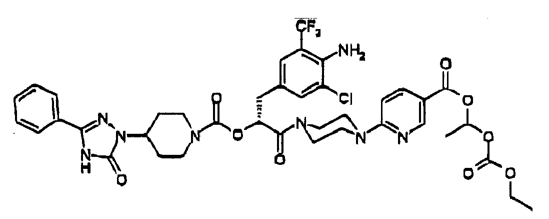 |
| (53) | 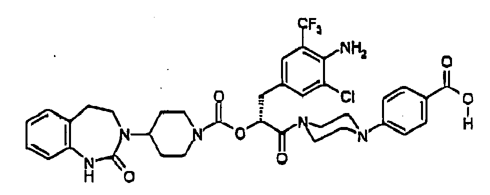 |
| (54) | 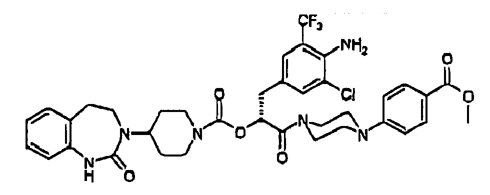 |
| (55) | 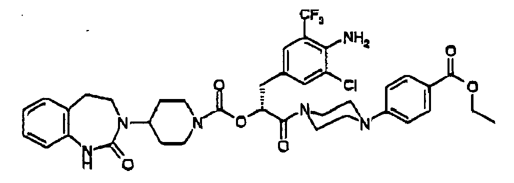 |
| (56) | 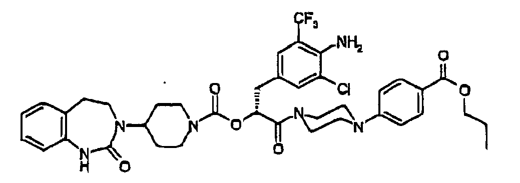 |
| (57) | 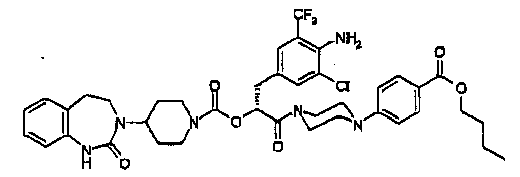 |
| (58) | 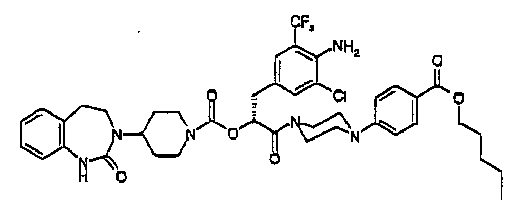 |
| (59) | 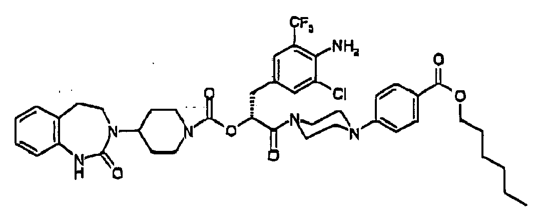 |
| (60) | 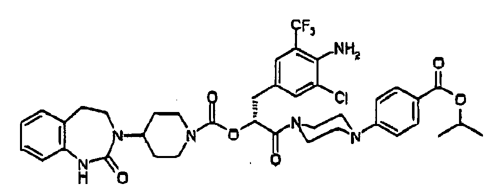 |
| (61) | 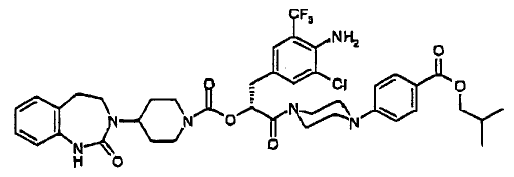 |
| (62) | 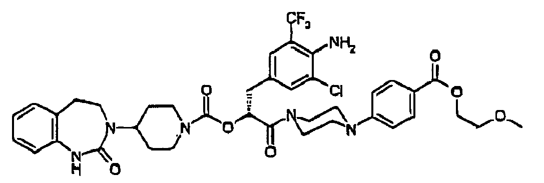 |
| (63) | 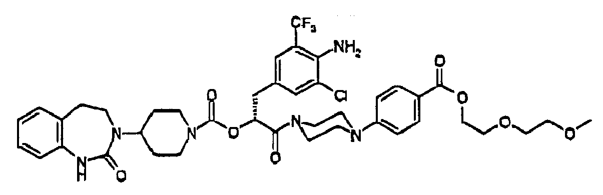 |
| (64) | 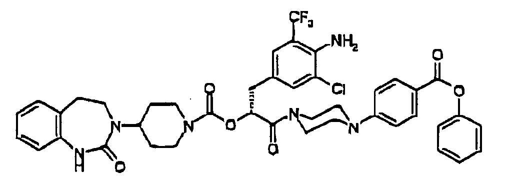 |
| (65) | 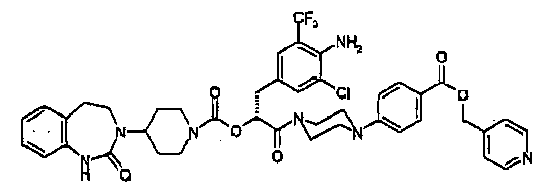 |
| (66) | 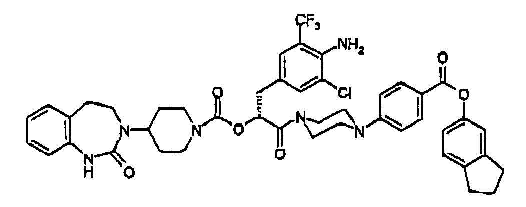 |
| (67) | 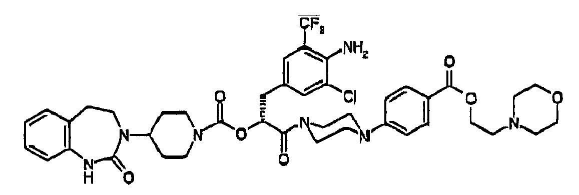 |
| (68) | 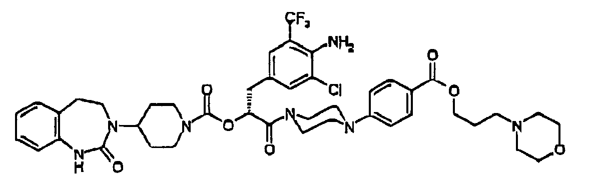 |
| (69) | 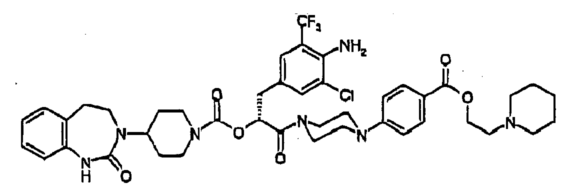 |
| (70) | 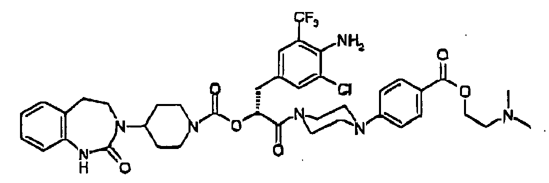 |
| (71) | 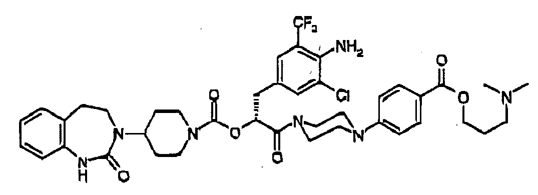 |
| (72) | 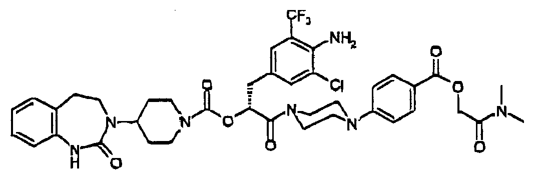 |
| (73) | 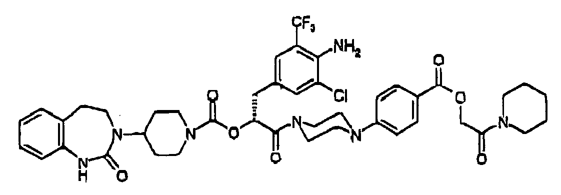 |
| (74) | 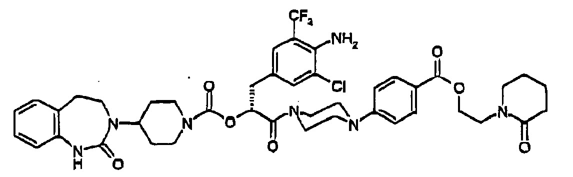 |
| (75) | 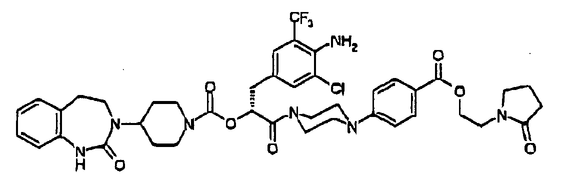 |
| (76) | 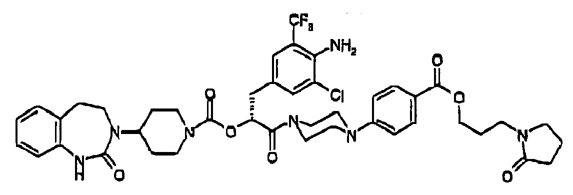 |
| (77) | 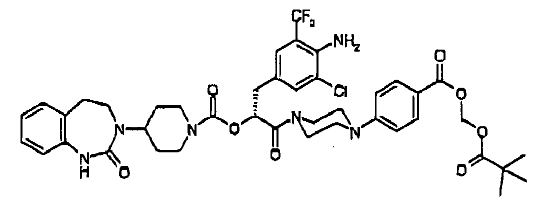 |
| (78) | 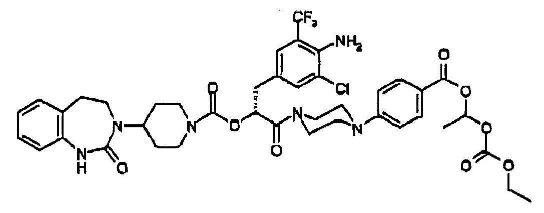 |
| (79) | 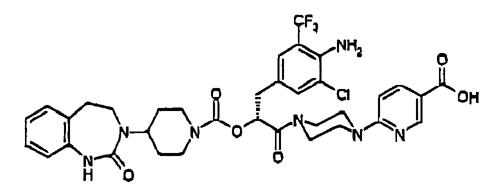 |
| (80) | 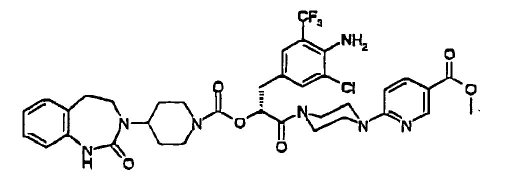 |
| (81) | 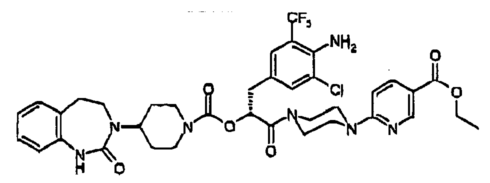 |
| (82) | 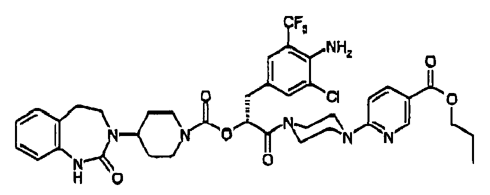 |
| (83) | 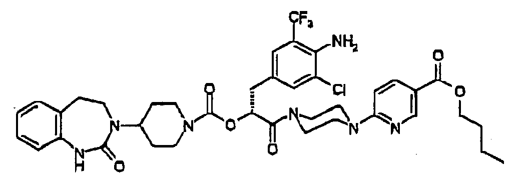 |
| (84) | 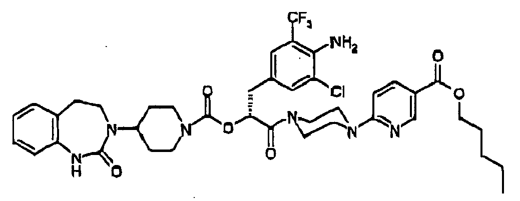 |
| (85) | 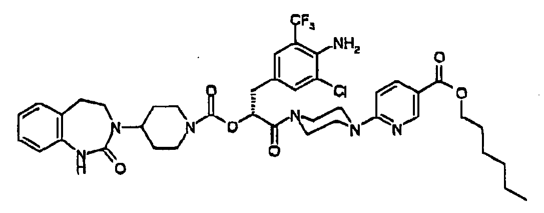 |
| (86) | 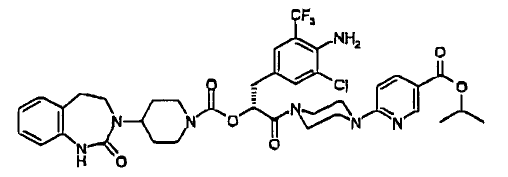 |
| (87) | 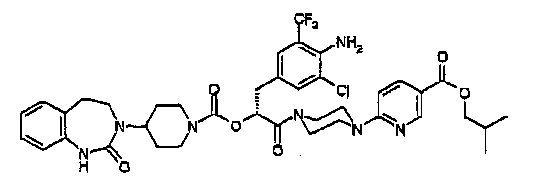 |
| (88) | 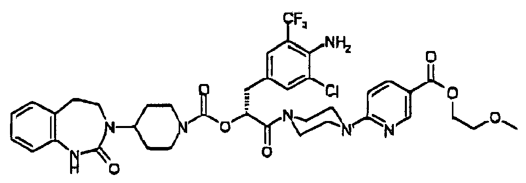 |
| (89) | 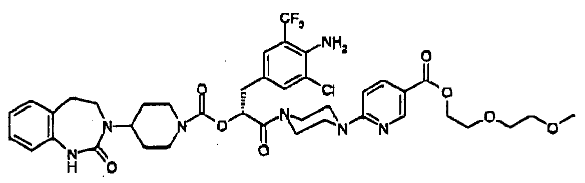 |
| (90) | 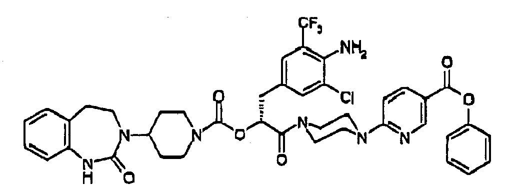 |
| (91) | 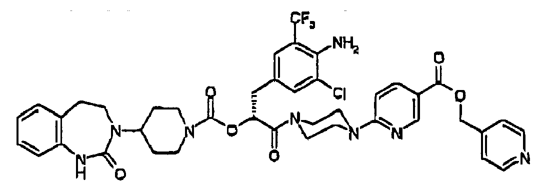 |
| (92) | 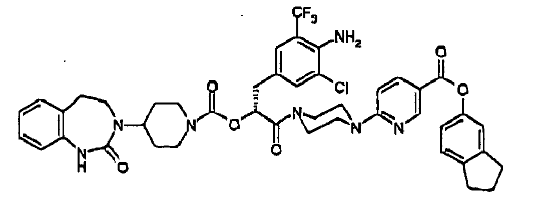 |
| (93) | 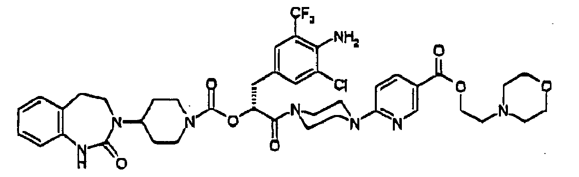 |
| (94) | 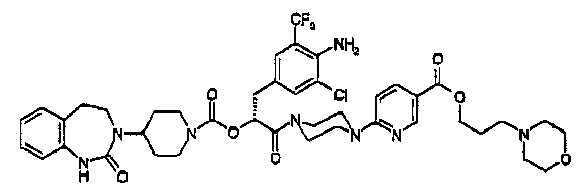 |
| (95) | 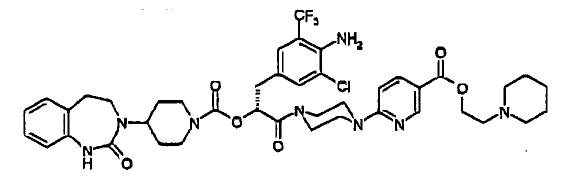 |
| (96) | 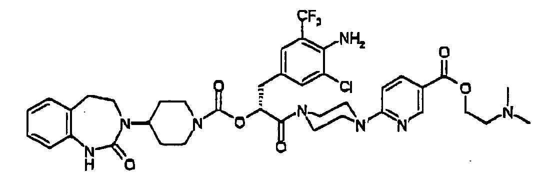 |
| (97) | 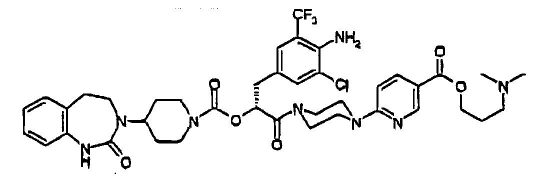 |
| (98) | 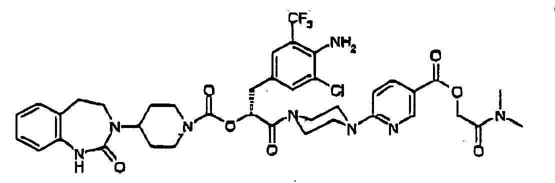 |
| (99) | 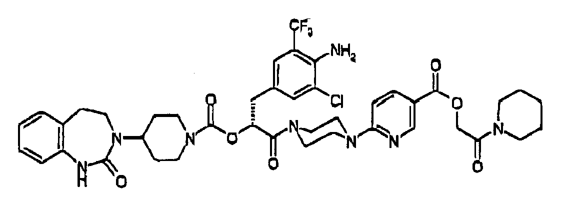 |
| (100) | 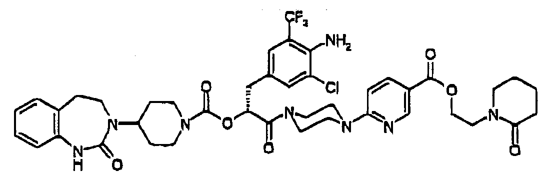 |
| (101) | 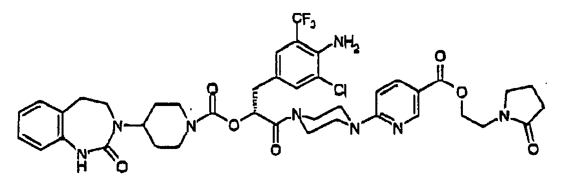 |
| (102) | 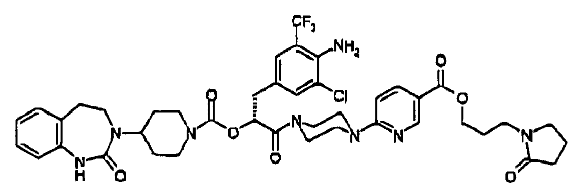 |
| (103) | 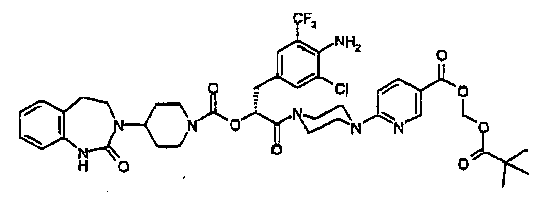 |
| (104) | 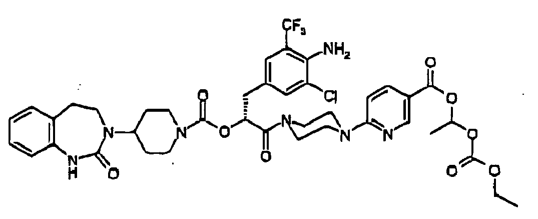 |
| (105) | 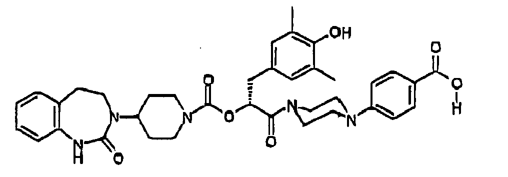 |
| (106) | 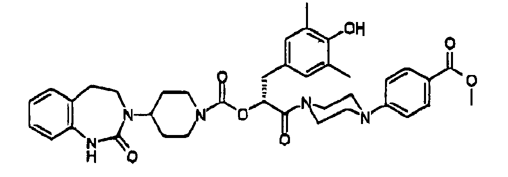 |
| (107) | 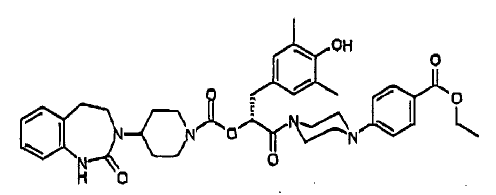 |
| (108) | 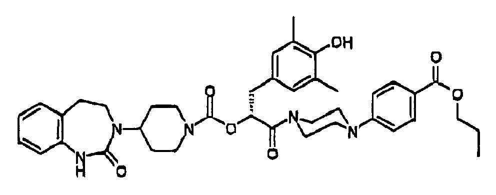 |
| (109) | 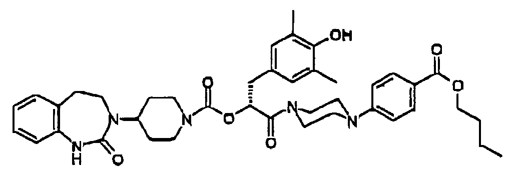 |
| (110) | 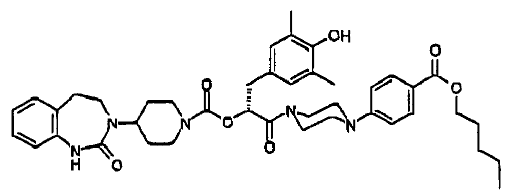 |
| (111) | 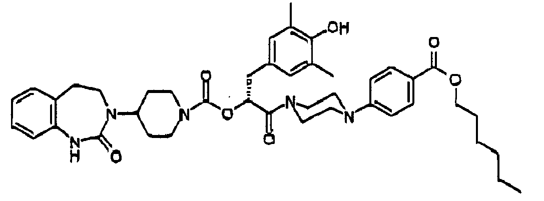 |
| (112) | 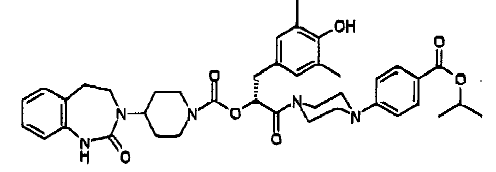 |
| (113) | 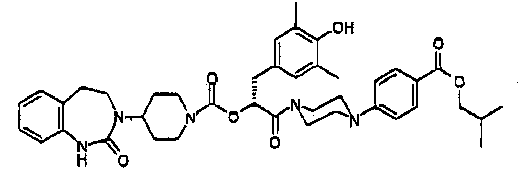 |
| (114) | 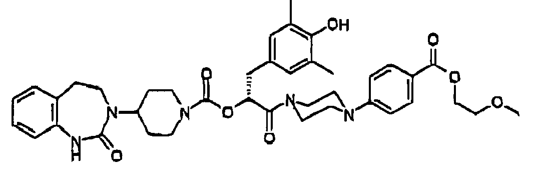 |
| (115) | 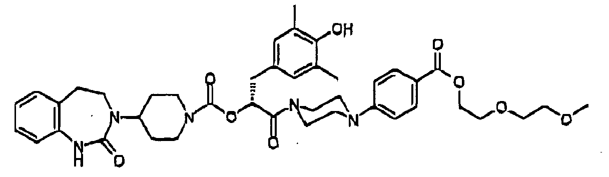 |
| (116) | 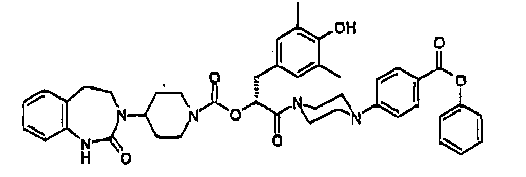 |
| (117) | 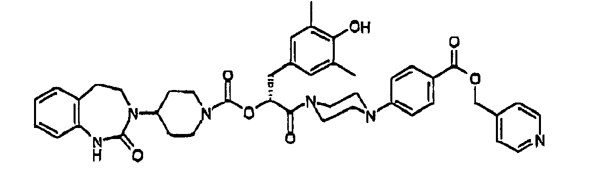 |
| (118) | 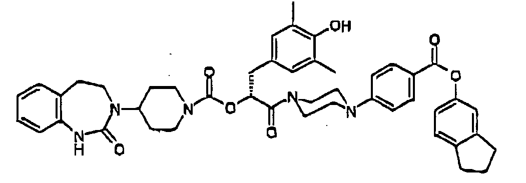 |
| (119) | 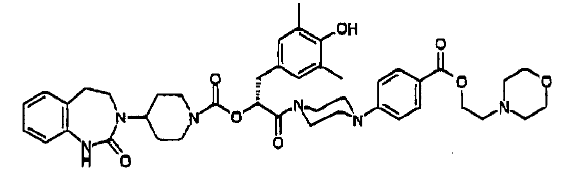 |
| (120) | 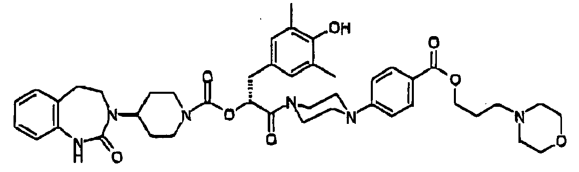 |
| (121) | 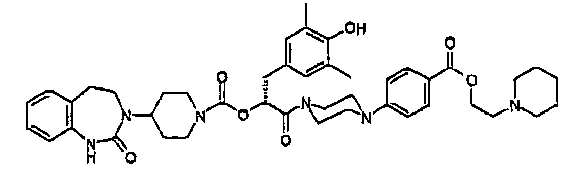 |
| (122) | 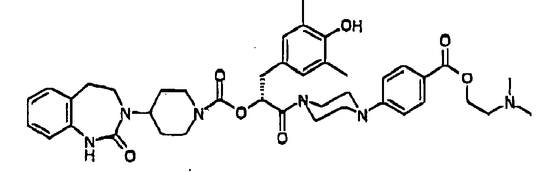 |
| (123) | 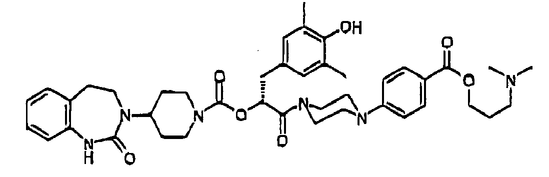 |
| (124) | 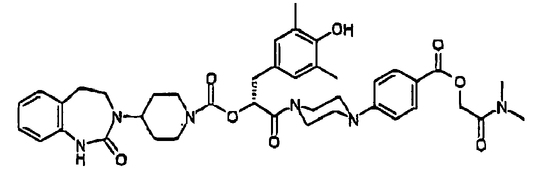 |
| (125) | 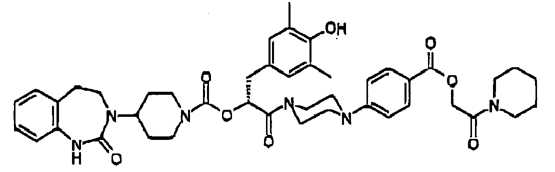 |
| (126) | 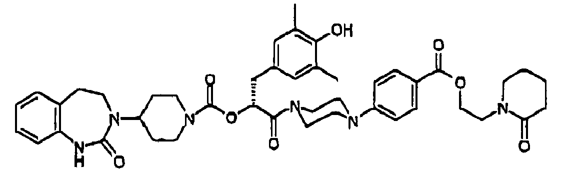 |
| (127) | 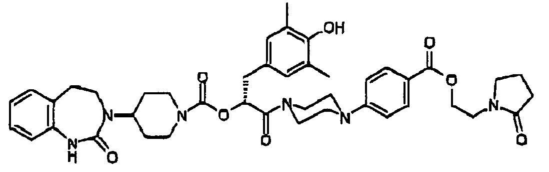 |
| (128) | 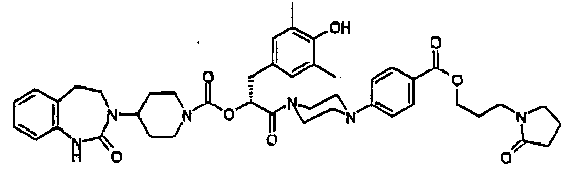 |
| (129) | 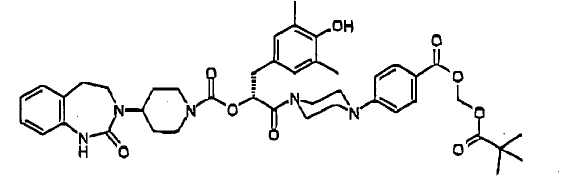 |
| (130) | 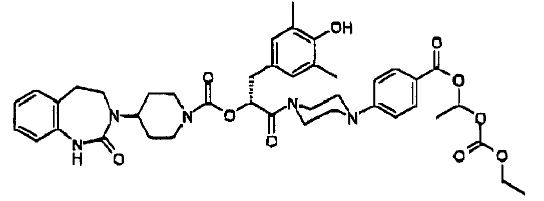 |
| (131) | 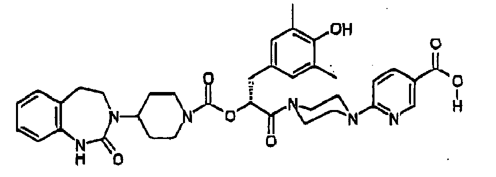 |
| (132) | 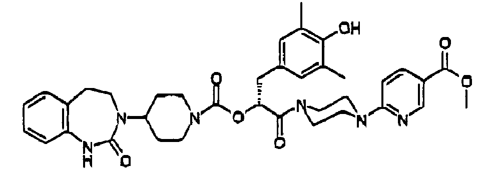 |
| (133) | 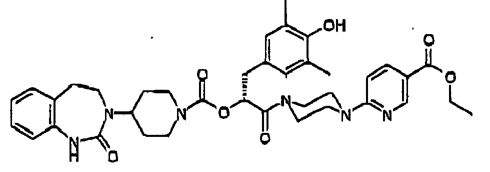 |
| (134) | 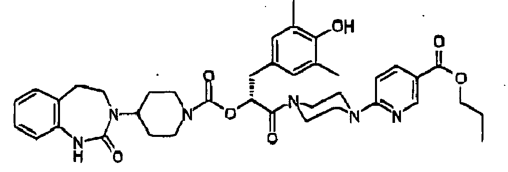 |
| (135) | 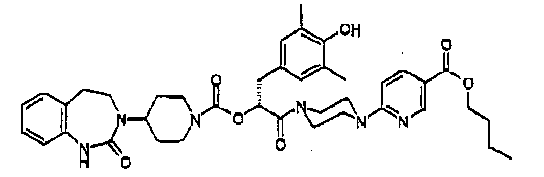 |
| (136) | 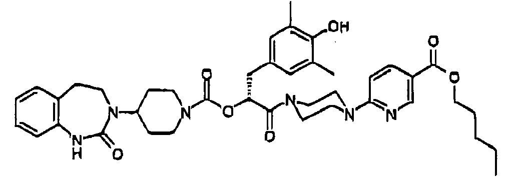 |
| (137) | 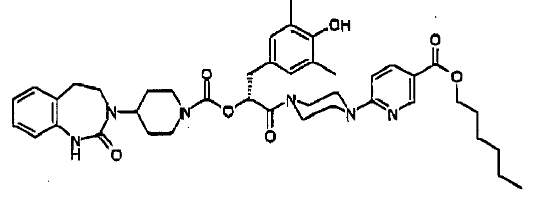 |
| (138) | 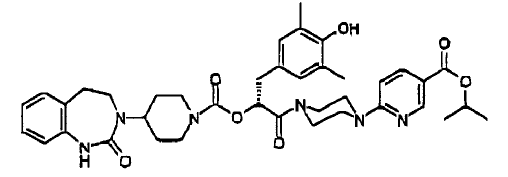 |
| (139) | 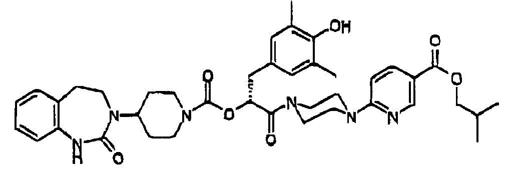 |
| (140) | 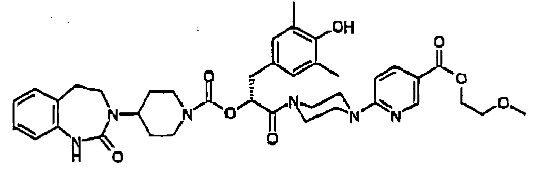 |
| (141) | 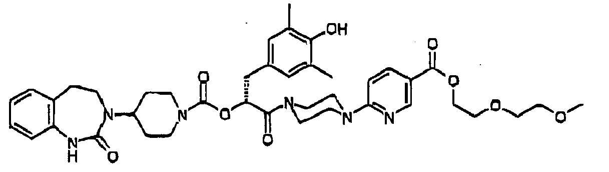 |
| (142) | 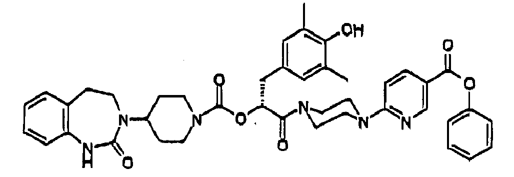 |
| (143) | 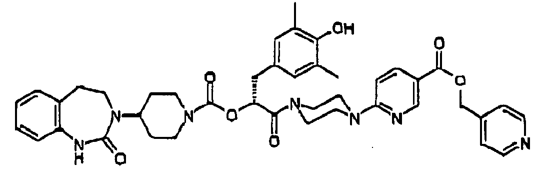 |
| (144) | 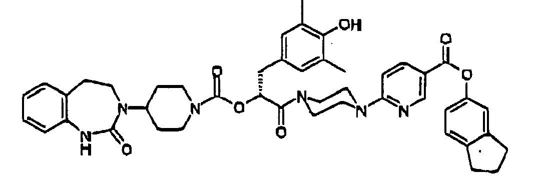 |
| (145) | 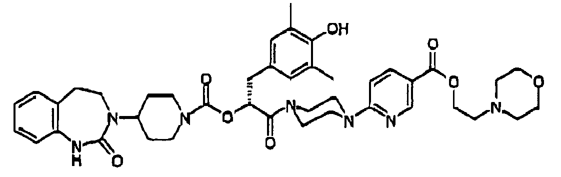 |
| (146) | 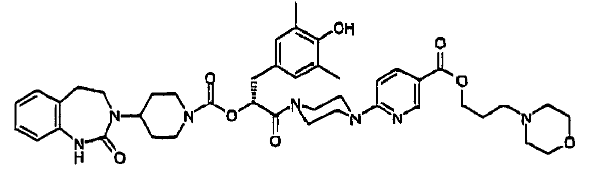 |
| (147) | 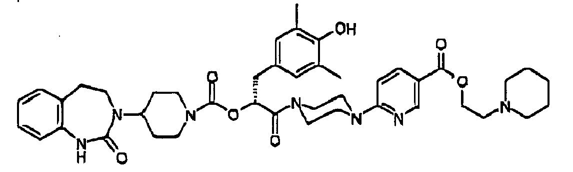 |
| (148) | 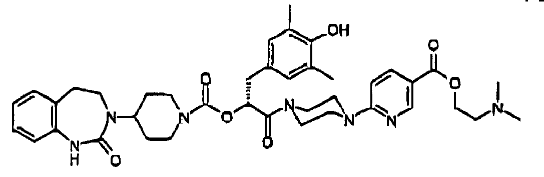 |
| (149) | 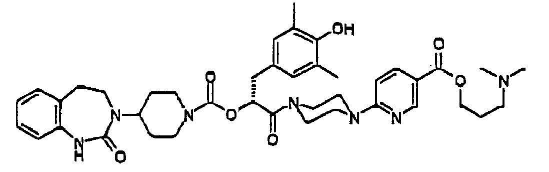 |
| (150) | 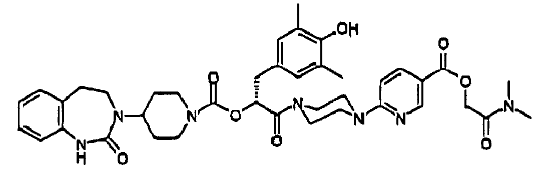 |
| (151) | 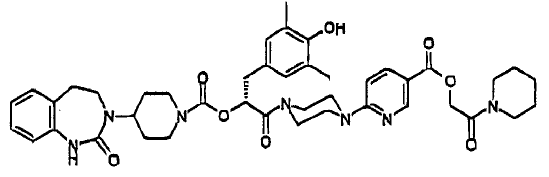 |
| (152) | 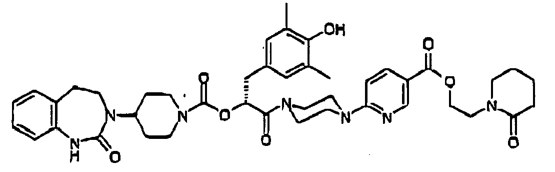 |
| (153) | 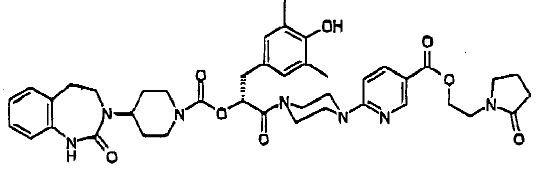 |
| (154) | 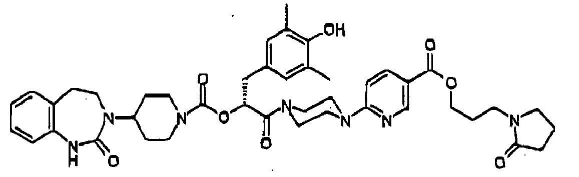 |
| (155) | 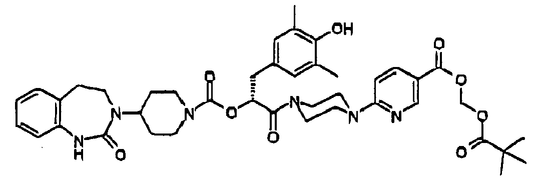 |
| (156) | 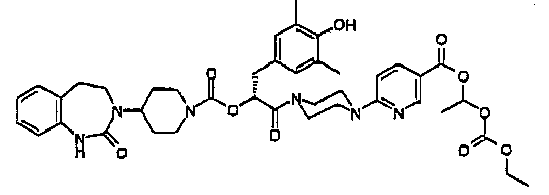 |
| (157) | 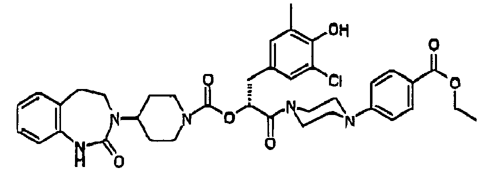 |
| (158) | 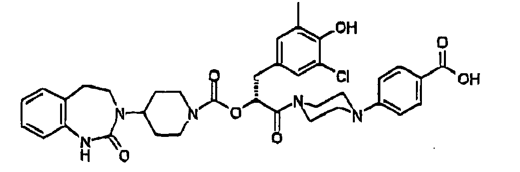 |
| (159) | 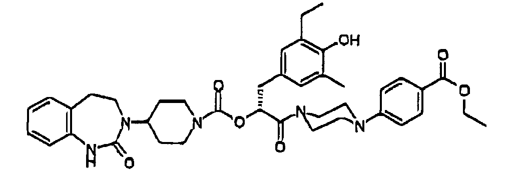 |
| (160) | 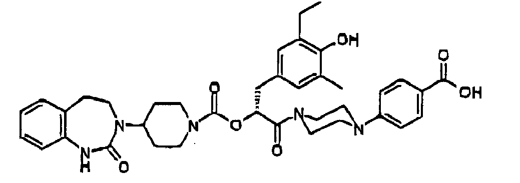 |
| (161) | 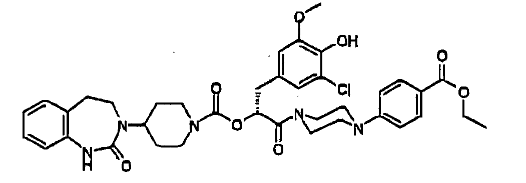 |
| (162) | 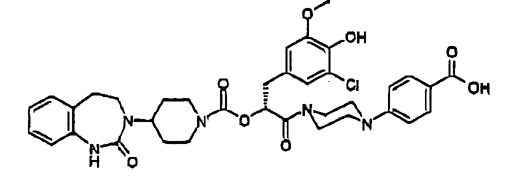 |
| (163) | 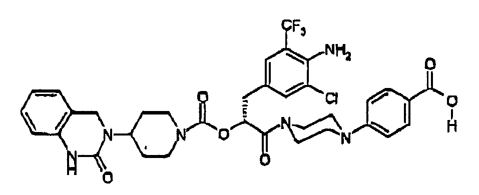 |
| (164) | 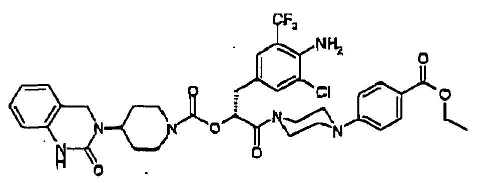 |
| (165) | 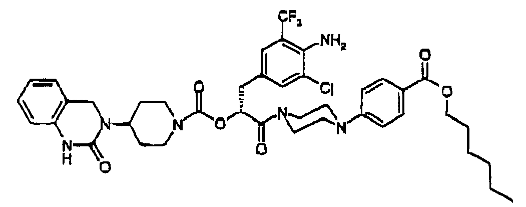 |
| (166) | 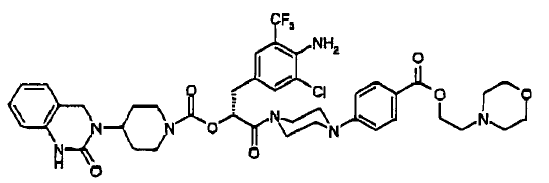 |
| (167) | 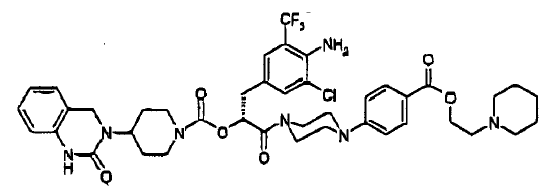 |
| (168) | 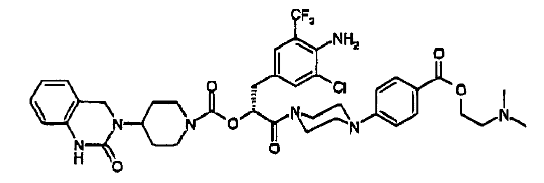 |
| (169) | 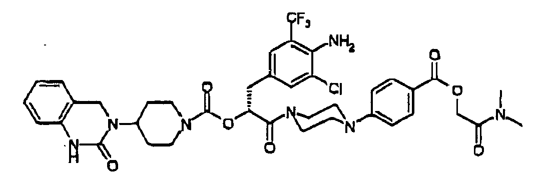 |
| (170) | 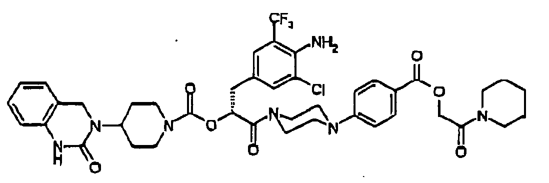 |
| (171) | 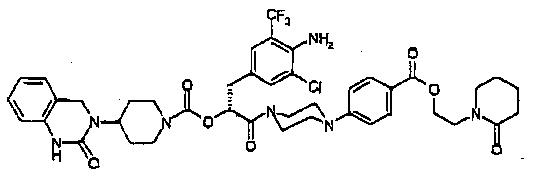 |
| (172) | 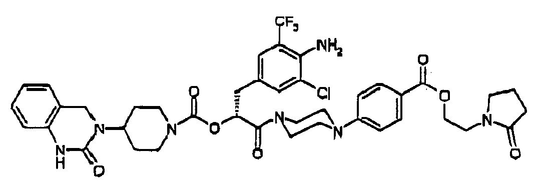 |
| (173) | 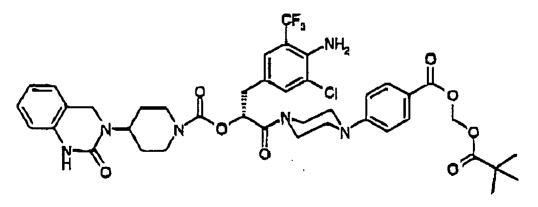 |
| (174) | 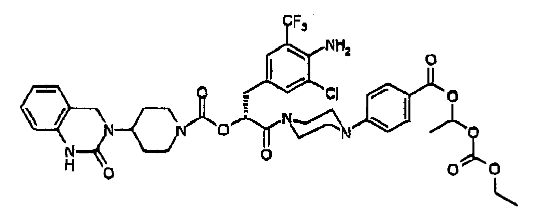 |
| (175) | 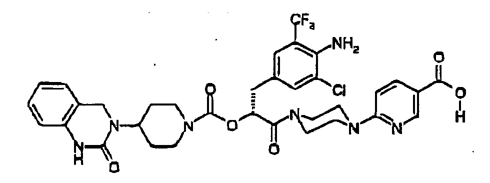 |
| (176) | 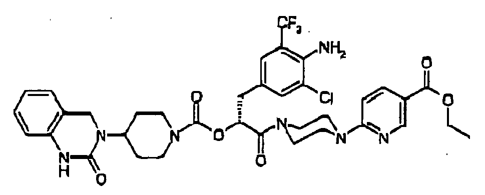 |
| (177) | 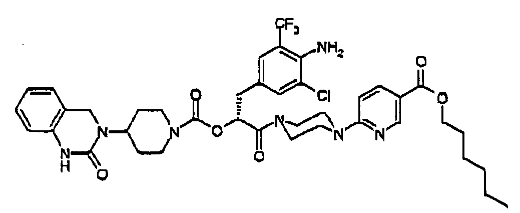 |
| (178) | 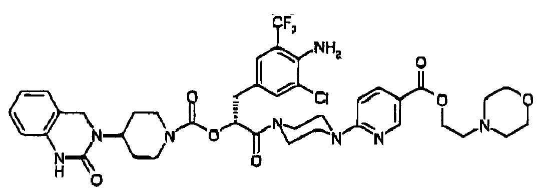 |
| (179) | 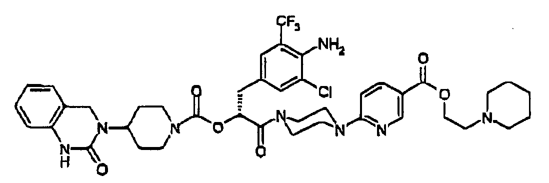 |
| (180) | 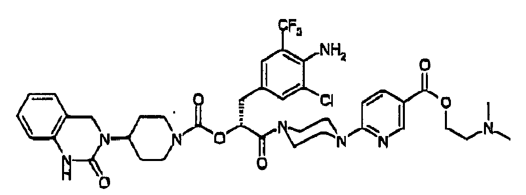 |
| (181) | 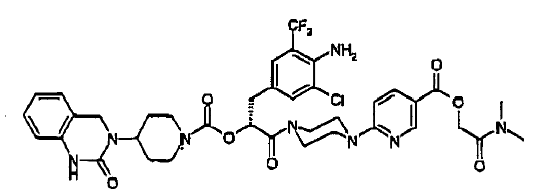 |
| (182) | 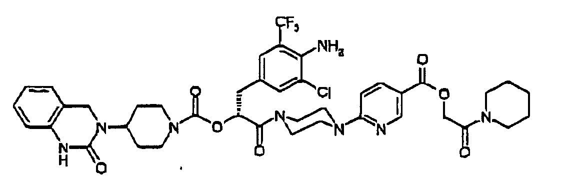 |
| (183) | 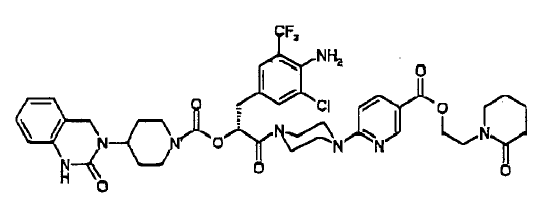 |
| (184) | 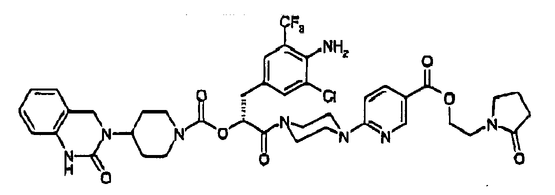 |
| (185) | 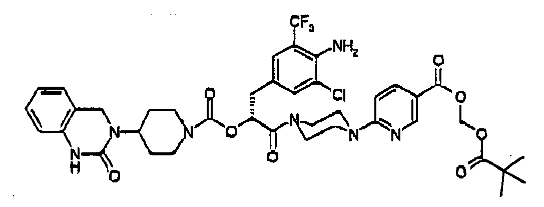 |
| (186) | 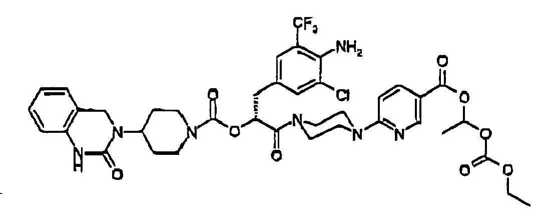 |
| (187) | 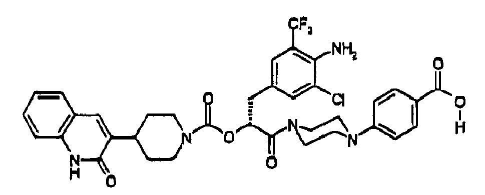 |
| (188) | 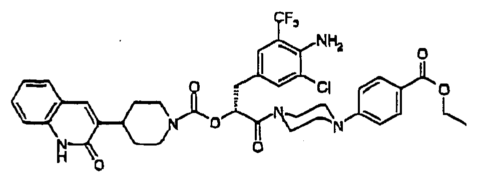 |
| (189) | 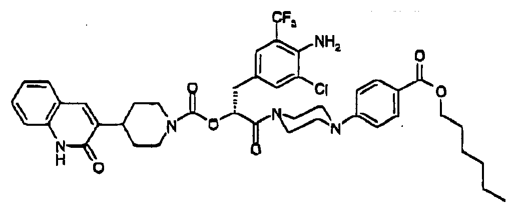 |
| (190) | 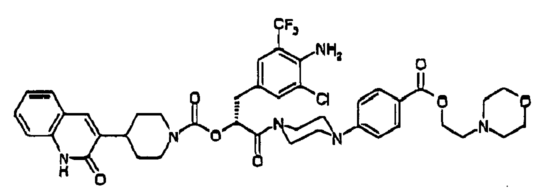 |
| (191) | 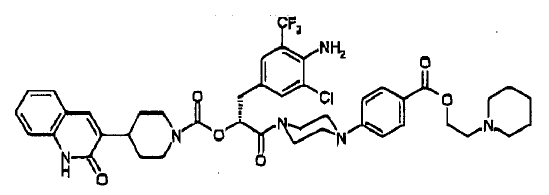 |
| (192) | 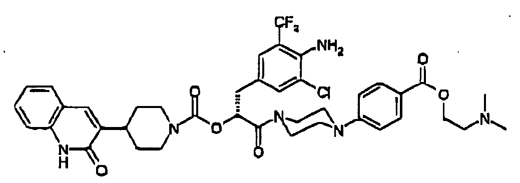 |
| (193) | 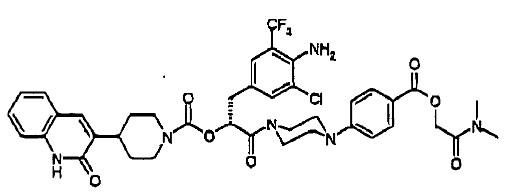 |
| (194) | 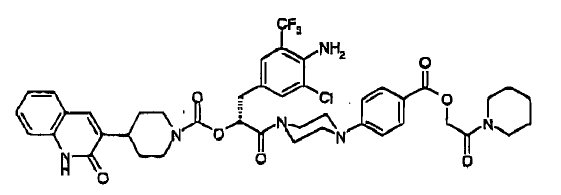 |
| (195) | 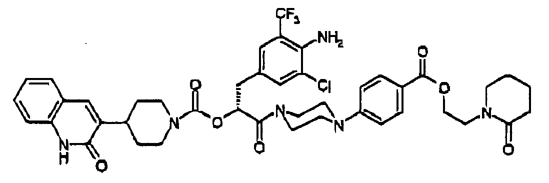 |
| (196) | 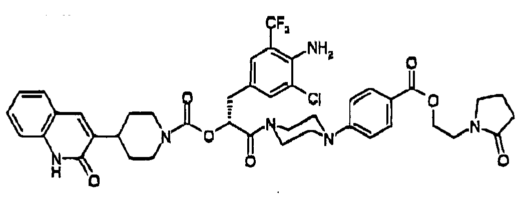 |
| (197) | 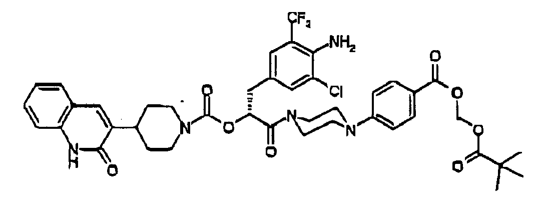 |
| (198) | 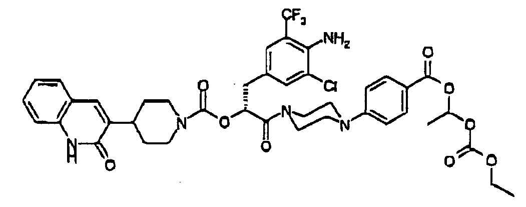 |
| (199) | 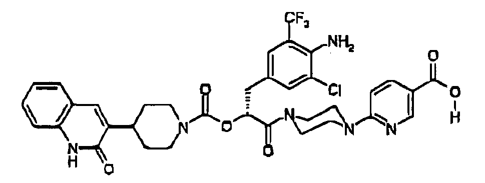 |
| (200) | 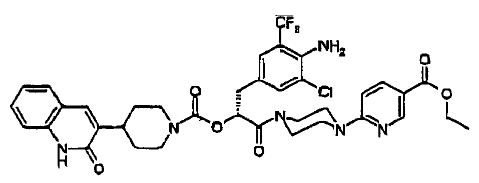 |
| (201) | 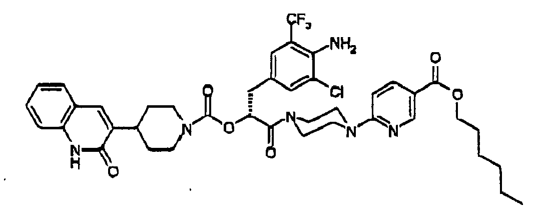 |
| (202) | 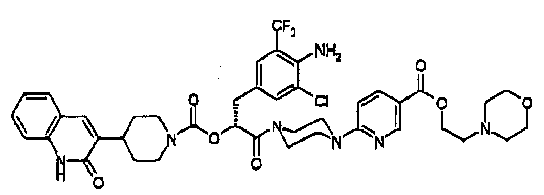 |
| (203) | 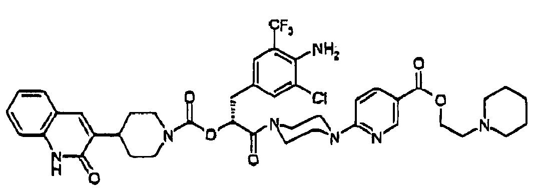 |
| (204) | 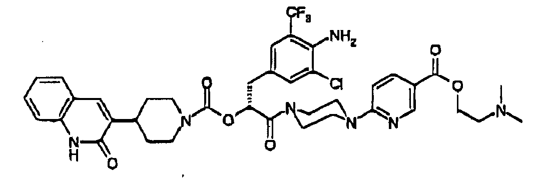 |
| (205) | 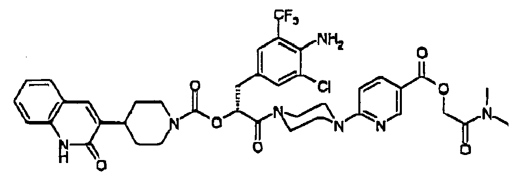 |
| (206) | 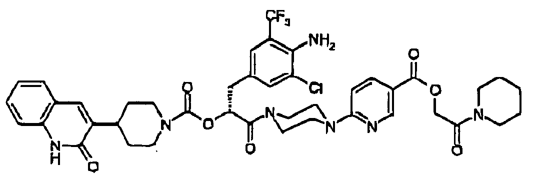 |
| (207) | 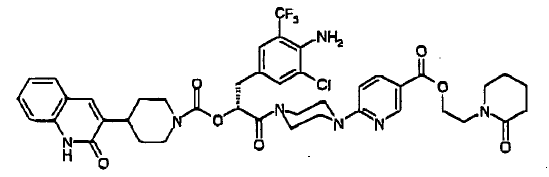 |
| (208) | 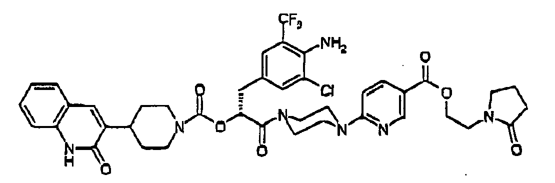 |
| (209) | 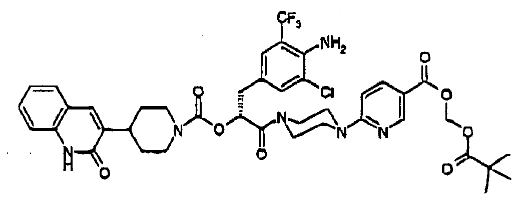 |
| (210) | 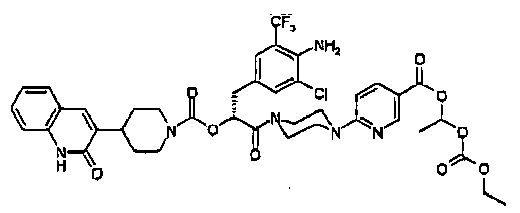 |
| (211) | 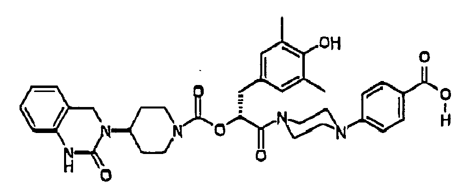 |
| (212) | 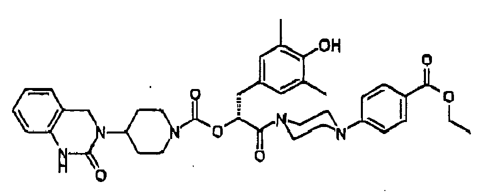 |
| (213) | 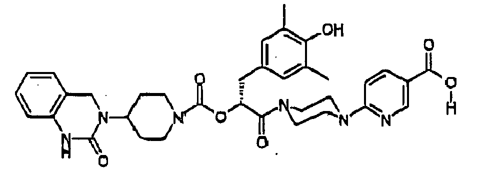 |
| (214) | 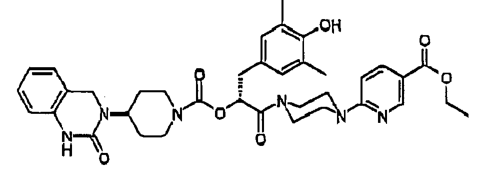 |
| (215) | 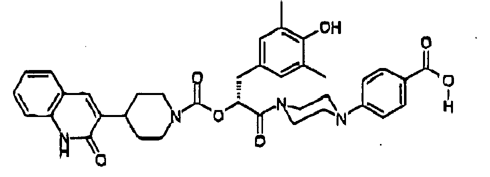 |
| (216) | 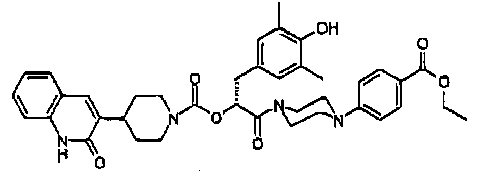 |
| (217) | 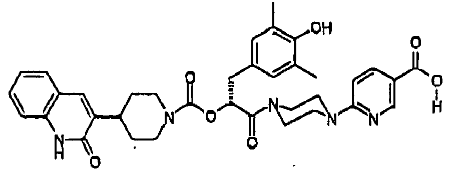 |
| (218) | 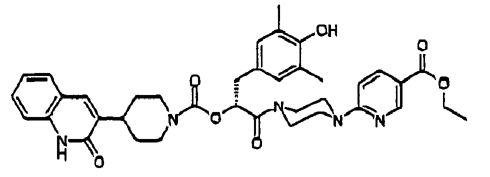 |

deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

### VERWENDETE BEGRIFFE UND DEFINTIONEN

Soweit nicht anders angegeben, sind alle Substituenten voneinander unabhängig. Sollten an einer Gruppe z.B. mehrere C₁₋₈-Alkylgruppen als Substituenten sein, so könnte im Fall von drei Substituenten C₁₋₈-Alkyl unabhängig voneinander einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird, falls vorhanden, ein Stem (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden verzweigte und unverzweigte Alkylgruppen mit 1 bis 3 Kohlenstoffatomen verstanden, unter dem Begriff "C₁₋₆-Alkyl" verzweigte und unverzweigte Alkylgruppen mit 1 bis 6 Kohlenstoffatomen und unter dem Begriff "C₁₋₈-Alkyl" verzweigte und unverzweigte Alkylgruppen mit 1 bis 8 Kohlenstoffatomen verstanden. Beispielsweise werden hierfür genannt: Methyl, Ethyl, *n*-Propyl, *iso*-Propyl, *n*-Butyl, *iso*-Butyl, *sec*-Butyl, *tert*-Butyl, *n*-Pentyl, *iso*-Pentyl, *neo*-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₃-Alkylen" (auch soweit sie Bestandteil anderer Reste sind) werden verzweigte und unverzweigte Alkylengruppen mit 1 bis 3 Kohlenstoffatomen und unter dem Begriff "C₂₋₄-Alkylen" verzweigte und unverzweigte Alkylengruppen mit 2 bis 4 Kohlenstoffatomen verstanden. Beispielsweise werden hierfür genannt: Methylen. Ethylen, Propylen, 1-Methylethylen, Butylen, 1-Methylpropylen, 1,1-Dimethylethylen, 1,2-Dimethylethylen. Sofern nicht anders beschrieben, umfassen die Definitionen Propylen und Butylen alle denkbaren isomeren Formen der gleicher Kohlenstoffanzahl, So umfasst beispielsweise Propylen auch 1-Methylethylen und Butylen umfasst 1-Methylpropylen, 1,1-Dimethylethylen, 1.2-Dimethylethylen.

"Halogen" steht im Rahmen der vorliegenden Erfindung für Fluor, Chlor, Brom oder Jod. Sofern nicht gegenteilig angegeben, gelten Fluor, Chlor und Brom als bevorzugte Halogene.

Verbindungen der allgemeinen Formel **I** können Säuregruppen besitzen, hauptsächlich Carboxylgruppen, und/oder basische Gruppen wie z.B, Aminofunktionen. Verbindungen der allgemeinen Formel **I** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, *p*-Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Wie vorstehend genannt, können die Verbindungen der Formel **I** in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel **I** mit anorganischen oder organischen Säuren vorliegen. Andererseits kann die Verbindung der Formel **I** im Falle von R gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Essigsäure, Fumarsäure. Bemsteinsäure, Milchsäure, Zitronensäure. Weinsäure oder Maleinsäure in Betracht. Ferner können Mischungen der vorgenannten Säuren eingesetzt werden. Zur Darstellung der Alkali- und Erdalkalimetallsalze der Verbindung der Formel **I** in der R Wasserstoff bedeutet, kommen vorzugsweise die Alkali- und ErdalKalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel **I** in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure. Maleinsäure, Methansulfonsäure, Milchsäure, Phosphorsäure, Salzsäure, Schwefelsäure, Weinsäure oder Zitronensäure in Betracht Ferner können Mischungen der vorgenannten Säuren eingesetzt werden,

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglykol- oder Methansulfonsäure

Die erfindungsgemäßen Verbindungen können als Racemate vorliegen, sofern sie nur ein Chiralitätselement besitzen, sie können aber auch als reine Enantiomere, d.h. in (*R*)- oder (*S*)-Form gewonnen werden. Bevorzugt sind Verbindungen die als Racemate bzw, als (*R*)-Form vorliegen.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen,

Gegenstand der Erfindung sind die jeweiligen Verbindungen gegebenenfalls in Form der einzelnen optischen lsomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### HERSTELLVERFAHREN

Die Verbindungen der allgemeinen Formel **I** werden nach prinzipiell bekannten Methoden hergestellt, Die folgenden Verfahren haben sich zur Herstellung der erfindungsgemäßen Verbindungen der allgemeinen Formel **I** besonders bewährt;
(a) Zur Herstellung von Verbindungen der allgemeinen Formel **I**, in der alle Reste wie eingangs erwähnt definiert sind:
   Kupplung einer Carbonsäure der allgemeinen Formel **IV** 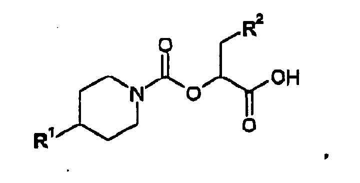 in der **R¹** und **R²** wie eingangs erwähnt definiert sind, mit einem Amin der allgemeinen Formel **V**

   **H-R³-R⁴-R⁵**

   in der **R³, R⁴** und **R⁵** wie eingangs definiert sind, wobei die Verknüpfung über das Stickstoffatom von **R³** erfolgt.
   Vor Durchführung der Reaktion können in den Resten des Amins der Formel **H-R³-R⁴-R⁵** gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach für den Fachmann geläufigen Methoden wieder abgespalten werden,
   Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Düsoprvpylcarbodümid (DIC) oder Ethyl-(3-dimethylamino-propyl)-rarbodilmid, O-(1*H*-Benzotriazol-1-yl)-*N,N-N',N'*-tetramethyluronium-hexa-fluorphosphat (HBTU) oder -tetr-afluorborat (TBTU) oder 1*H*-Benzotriazol-1-yl-oxy-tris-(dimethylamino)-phosphanium-hexafluorphosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran, Acetonitril. Dimethylformamid (DMF), Dimethylacetamid (DMA), *N*-Methylpyrrolidon (NMP) oder Gemischen aus diesen und bei Temperaturen zwischen -30°C und +30°C, bevorzugt -20°C und +25°C, durchgeführt. Sofern erforderlich wird als zusätzliche Hilfsbase *N*-Ethyldiisopropylamin (Hünig-Base) bevorzugt.
   Als weiteres Kupplungsverfahren zur Synthese von Verbindungen der allgemeinen Formel **I** wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel V und dem Kohlensäuremonoisobutylester erhalten wird. Die Herstellung dieses gemischten Anhydrids und die Kupplung mit den Aminen der allgemeinen Formel **VI** erfolgt im Eintopfverfahren unter Verwendung der voranstehend genannten Lösungsmittel und bei Temperaturen zwischen -20°C und +25°C. bevorzugt zwischen 0°C und +25°C.
(b) Zur Herstellung von Verbindungen der allgemeinen Formel **I,** in der alle Reste wie eingangs erwähnt definiert sind:
   Kupplung einer Verbindung der allgemeinen Formel **VI** 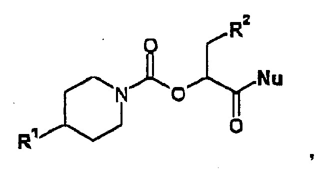 in der **R¹** und **R²** wie eingangs erwähnt definiert sind und **Nu** eine Austrittsgruppe, beispielsweise ein Halogenatom, wie das Chlor-, Brom- oder lodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch eine oder zwei Methylgruppen im Kohlenstoffgerüst substituierte 1*H*-Pyrazol-1-yl-, eine 1*H*-1,2,4-Triazol-1-yl-, 1*H*-1,2,3-Thazol-1-yl-, 1*H*-1,2,3,4-Tetrazol-1-yl-, eine Vinyl-, Propargyl-, *p*-Nitrophenyl-, 2,4-Dinitrophenyl-, Trichlorphenyl-, Pentachlorphenyl-, Pentafluorphenyl-, Pyranyl- oder Pyridinyl-, eine Dimethylaminyloxy-, 2(1*H*)-Oxopyridin-1-yl-oxy-, 2,5-Dioxopyrrolidin-1-yloxy-, Phthalimidyloxy-, 1*H*-Benzotriazol-1-yloxy- oder Azidgruppe darstellt, mit einem Amin der allgemeinen Formel **V**

   **H-R³-R⁴-R⁵ ,**

   in der alle Reste wie eingangs erwähnt definiert sind und wobei die Verknüpfung über das Stickstoffatom des Amins R³ erfolgt.

Vor Durchführung der Reaktion können in den Resten des Amins der allgemeinen Formel **V** gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach für den Fachmann geläufigen Methoden wieder abgespalten werden.

Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C, und gegebenenfalls in Gegenwart von Lösungsmitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, *N*-Ethyldiisopropylamin, *N*-Ethyldicyclohexylamin, 1,4-Di-azabicyclo[2,2,2]octan oder 1,8-Diaza-bicyclo[5,4,0]undec-7-en, als Lösungsmittel beispielsweise Dichlormethan, Tetrahydrofuran, 1,4-Dioxan, Acetonitril, Dimethylformamid, Dimethylacetamid, *N*-Methylpyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.

Die erfindungsgemäßen neuen Verbindungen der allgemeinen Formel **I** enthalten ein oder mehrere Chiralitätszentren, Sind beispielsweise zwei Chiralitätszentren vorhanden, dann können die Verbindungen in Form zweier diastereomerer Antipodenpaare auftreten. Die Erfindung umfasst die einzelnen isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel **I** fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (*R*)-(+)-1-Phenylethylamin, (*S*)-(-)-1-Phenylethylamin oder (*S*)-Brucin, entstehen.

Nach einem üblichen Verfahren zur Isomerentrennung wird das Racemat einer Verbindung der allgemeinen Formel **I** mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol. Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (*R*)- oder (*S*)-Enantiomer bzw. ein Gemisch zweier optisch aktiver, unter die allgemeine Formel **I** fallender, diastereomerer Verbindungen wird auch dadurch erhalten, dass man die oben beschriebenen Synthesen mit jeweils einer geeigneten (*R*)- bzw. (*S*)-konfigurierten Reaktionskomponente durchführt,

Die als Ausgangsverbindungen benötigten Hydroxycarbonsäuren der allgemeinen Formel V sind durch Umsetzung von Piperidinen der allgemeinen Formel **VII** 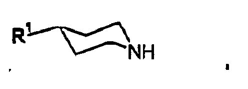 in der **R¹** wie eingangs erwähnt definiert ist, mit Kohlensäurederivaten der allgemeinen Formel **VIII** 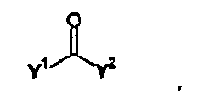 in der **Y¹** und **Y²** nucleofuge Gruppen bedeuten, die gleich oder verschieden sein können, bevorzugt das Chloratom, die *p*-Nitrophenoxy- oder Trichlormethoxy-Gruppe,
und mit Verbindungen der allgemeinen Formel **IX** 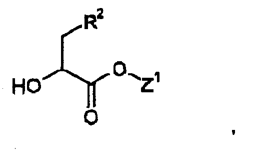 in der **R²** wie eingangs erwähnt definiert ist und **Z¹** eine Schutzgruppe für eine Carboxygruppe darstellt, beispielsweise eine C₁₋₆-Alkyl- oder eine gegebenenfalls substituierte Benzylgruppe, wobei die Alkylgruppen linear oder verzweigt sein können und die Benzylgruppe durch ein oder zwei Methoxygruppen substituiert sein kann, erhältlich. Bevorzugt ist für **Z¹** die Methyl-, Ethyl-, *tert*-Butyl oder Benzylgruppe. Vor Durchführung der Reaktion kann im Rest **R²** einer Verbindung der Formel (VI) gegebenenfalls vorhandene Hydroxyfunktionen durch übliche Schutzreste geschützt und gegebenenfalls verwendete Schutzreste nach Durchführung der Reaktion nach dem Fachmann geläufigen Methoden wieder abgespalten werden.

In einer ersten Stufe werden die Verbindungen der allgemeinen Formel **VII** in einem Lösungsmittel, beispielsweise in Dichlormethan, THF, Pyridin oder deren Mischungen, bei einer Temperatur zwischen -20°C bis 50°C in Gegenwart einer Base, beispielsweise Triethylamin, Pyridin oder Ethyldiisopropylamin, mit den Kohlensäurederivaten der allgemeinen Formel **VIII** zur Reaktion gebracht. Die dabei entstehende Zwischenstufe kann aufgereinigt oder ohne Reinigung weiter umgesetzt werden. Die Umsetzung dieser Zwischenstufen mit Verbindungen der allgemeinen Formel **IX** erfolgt ebenfalls in einem der oben genannten Lösungsmittel, und bei den oben genannten Temperaturen, in Gegenwart einer Base, wie Triethylamin oder Pyridin, mit oder ohne Zusatz eines Aktivierungsreagenz, wie z.B, 4-Dimethylaminopyridin. Zur Aktivierung können die Verbindungen der allgemeinen Formel **IX** auch mittels eines Metallhydrides, wie z.B. NaH oder KH, deprotoniert werden, wobei in diesem Fall auf die Gegenwart der Base oder des Aktivierungsreagenzes verzichtet werden kann.

Die Ausgangsverbindungen der Formel **VII** und **VIII** sind entweder käuflich, literaturbekannt oder können nach literaturbekannten Methoden hergestellt werden.

Ein Zugang zu Verbindungen der allgemeinen Formel **IX** besteht in der Umsetzung von Aldehyden der allgemeinen Formel **X** 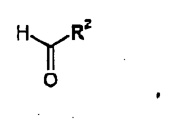 in der **R²** wie eingangs erwähnt definiert ist, mit *N*-Acetylglycin in Acetanhydrid als Lösungsmittel in Gegenwart von Alkaliacetat, bevorzugt Natrium- oder Kaliumacetat, bei geeigneter Temperatur, bevorzugt bei 80 bis 130°C.
Die primär entstehenden Azlactone werden ohne Isolierung zu den Verbindungen der allgemeinen Formel **XI** 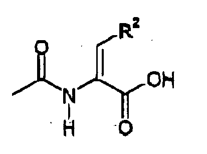 in der R² wie eingangs erwähnt definiert ist, hydrolysiert. Durch weitere Umsetzung in Gegenwart von wässrigen Mineralsäuren, wie beispielsweise Schwefel-, Phosphor- oder Chlorwasserstoffsäure, bevorzugt jedoch von Chlorwasserstoffisäure, werden Verbindungen der allgemeinen Formel **XII** 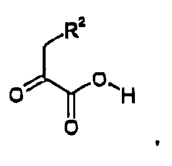 in der **R²** wie eingangs erwähnt definiert ist, erhalten.
Diese werden dann mit geeigneten Reduktionsmittelri in die Verbindungen der allgemeinen Formel **XIII** 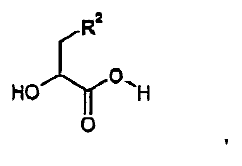 in der **R²** wie eingangs erwähnt definiert ist, überführt.
Als Reduktionsmittel können Alkaliborhydride, wie Natrium- oder Kaliumborhydrid verwendet werden. Weitere Reduktionsmittel stellen Chlordialkylborane, wie Chlordicyclohexylboran, dar. Werden chirale Chlordialkylborane, wie z.B. B-Chlordiisopinocampheylboran benutzt, können die Verbindungen der allgemeinen Formel **XIII** in enantiomerenreiner Form isoliert werden. Die weitere Umsetzung von Verbindungen der allgemeinen Formel **XIII** zu Verbindungen der allgemeinen Formel **IX** erfolgt im alkoholischen Milieu, bevorzugt in Methanol oder Ethanol, in Gegenwart einer geeigneten Säure, wie Chlorwasserstoffsäure. Die Reaktion kann alternativ durch Umsetzung in alkoholischen Lösungsmitteln, bevorzugt Methanol, mit Thionychlorid erfolgen.

Alle Verbindungen der allgemeinen Formel **I,** die primäre oder sekundäre Amino-, Hydroxy- oder Hydroxycarbonylfuhktionen enthalten, werden bevorzugt aus mit Schutzgruppen versehenen Vorstufen gewonnen. Als Schutzgruppen für Aminofunktionen kommen beispielsweise eine Benzytoxycarbonyl-, 2-Nitrobenzyloxycarbonyl-, 4-Nitrobenzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5-Dimethoxy-α,α-dimethyl-beneoxycarbonylgruppe, eine Alkoxycarbonylgruppe mit insgesamt 1 bis 5 Kohlenstoffatomen im Alkylteil, beispielsweise die Methoxycarbonyl-, Ethoxycarbonyl-, *n*-Propoxycarbonyl-, Isopropoxycarbonyl-, n-Butoxycarbonyl-, 1-Methylpropoxycarbonyl-, 2-Mathylpropoxy-carbonyl- oder *tert*-Butyloxycarbonylgruppe, die Allyloxycarbonyl-, 2,2,2-Trichlor-(1,1-dimethylethoxy)carbonyl- oder 9-Fluorenylrnethoxycarbonyl-Gruppe oder eine Formyl-, Acetyl- oder Trifluoracetylgruppe in Frage.
Als Schutzgruppe für Hydroxyfunktionen kommt beispielsweise eine Trimethylsilyl-. Triethylsilyl-, Triisopropyl-, *tert*-Butyldirmethylsilyl- oder *tert*-Butyldiphenylsilylgruppe, eine *tert*-Butyl-, Benzyl-, 4-Methoxybenzyl- oder 3,4-Dimethoxybenzylgruppe in Frage.
Als Schutzgruppe für Hydroxycarbonylfunktionen kommt beispielsweise eine Alkylgruppe mit insgesamt 1 bis 5 Kohlenstoffatomen, beispielsweise die Methyl-, Ethyl-, *n*-Propyl-.

Isopropyl-, *n*-Butyl-, *tert*-Butyl, Allyl-, 2,2,2-Trichlorethyl-, Benzyl- oder 4-Methoxybenzylgruppe in Frage.

Die erhaltenen Verbindungen der allgemeinen Formel **I** können, sofern sie geeignete basische Funktionen enthalten, insbesondere für pharmazeutische Anwendungen in ihre physiologisch verträglichen Salze mit anorganischen oder organischen Säuren übergeführt werden. Als Säuren kommen hierfür beispielsweise Salzsäure, Bromwasserstoffsäure, Phosphorsäure, Salpetersäure, Schwefelsäure, Methansulfonsäure, Ethansulfonsäure, Benzolsulfonsäure, *p*-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die neuen Verbindungen der Formel **I**, falls sie Carbonsäurefunktionen enthalten, in ihre Additionssalze mit anorganischen oder organischen Basen, insbesondere für die pharmazeutische Anwendung in ihre physiologisch verträglichen Additionssalze überführen, Als Basen kommen hierfür beispielsweise Natriumhydroxid, Kaliumhydroxid, Ammoniak. Cyclohexylamin, Dicyclohexylamin, Ethanolamin, Diethanolamin und Triethanolamin in Betracht.

Die vorliegende Erfindung betrifft Racemate, sofern die Verbindungen der allgemeinen Formel **I** nur ein Chiralitätselement besitzen. Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enantiomeren, aus denen sich die erwähnten Racemate zusammensetzen.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Die neuen Verbindungen der allgemeinen Formel **I** und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zeiten

SK-N-MC-Zellen werden in "Dulbecco's modified Eagle Medium" kultiviert. Das Medium konfluenter Kulturen wird entfernt, Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert, Nach Resuspension in 20 ml "Balanced Safts Solution" [BSS (in mM): NaCl 120, KCl 5.4, NaHCO₃ 16.2, MgSO₄ 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1% Rindersenrm-Albumin und 0.1% Bacitracin, rezentrifugiert und resuspendiert (1 ml/1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM ¹²⁵I-Iodotyrosyl-Caicitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1 %) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test IC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei -20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagvnistische Eigenschaften in einem Dosisbereich zwischen 10⁻¹² bis 10⁻⁵ M.

### INDIKATIDNSGEBIETE

Aufgrund ihrer phannakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie spannungskopfschmerzen. Weiterhin beeinflussen die erfindungsgemäßen Verbindungen auch die folgenden Erkrankungen positiv:
Nicht-insulinabhängigen Diabetes mellitus ("NIDDM"), cardiovaskulfire Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), gerleralisierter Weichteilrheumatismus (Fibromyalgie), neurogene Entzündungen der oralen Mucosa, entzündliche Lungenericrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0,01 bis 10 mg/kg Körpergewicht, vorzugsweise 0.1 bis 10 mg/kg Körpergewicht, jeweils 1 bis 3 x täglich. Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoftklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosterolden, Calcium-Antagonisten. 5-HT_{1B/1D}-Agonisten oder andere Antimigränemittein, die zusammen mit einem oder mehreren inerten üblichen Trägerstoffen und/oder Verdünnungsmitteln, z.B. mit Maisstärke, Milchzucker. Rohrzucker, mikrokristalliner Zellulose. Magnesiumstearat, Polyvinylpyrrolidon, Zitronensäure, Weinsäure, Wasser, Wasser/Ethanol, Wasser/-Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können,

Für die oben erwähnten Kombinationen Kommen somit als weitere Wirksubstanzen beispielsweise die nicht-steroldalen Antiphlogistika Aceclofenac, Acemetacin, Acetylsalicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EPZ-Rezeptor Antagonisten und IP-Rezeptor Antagonisten.

Weiterhin können Ergotamin, Dihydroergotamin. Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden,

Außerdem können CGRP-Antagonisten mit Vanilloid-Rezeptor Antagonisten, wie z.B. VR-1 Antagonisten, Glutamafirezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B, P2X3 Antagonisten, NO-Synthase Inhibitoren, wie z.B. iNOS Inhibitoren, Calciumkanal-Blockem, wie z.B. PQ-typ Blockern, N-typ Blockern, Källumkenalöffnern, wie z.B. KCNQ Kanalöffnern, Natriumkanal Blockern, wie z.B. PN3 Kanal Blockern, NMDA-Rezeptor Antagonisten, Acid-sensing lonenkanal Antagonisten, wie z,B, ASIC3 Antagonisten, Bradykinin Rezeptor Antagonisten wie z.B. B1-Rezeptor Antagonisten, Canriabinoid-Rezeptoren Agonisten, wie z.B. CB2 Agonisten, CB1 Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosolformulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0,5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **I** gemäß der obigen bevorzugten Ausführungsformen.

Besonders bevorzugt ist es, wenn die Verbindungen der Formel **I** oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin. Cyclamat, Glycerin oder Zucker sowie ein Geschmack verbessemdes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägem, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z,B, hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicatciumphosphat zusammen mit verschiedenen Zuschlagstoffen, wie Stärke, vorzugsweise Kartoffelstärke. Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserem oder Farbstoffen versetzt werden.

Ebenfalls bevorzugt ist es, wenn die Verbindungen der Formel **I** inhalativ verabreicht werden, besonders bevorzugt ist es, wenn die Verabreichung ein oder zweimal täglich erfolgt, Hierzu müssen die Verbindungen der Formel **I** in inhalierbaren Darreichungsformen bereitgestellt werden. Als inhalierbare Darreichungsformen kommen Inhalationspulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalationspulver

Sind die Verbindungen der Formel **I** im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose. Maltose), Oligo- und Polysaccharide (z.B. Dextrane). Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhalationspulver durch Mahlen und Mikronisieren sowie durch abschließendes Mischen der Bestandteile sind aus dem Stand der Technik bekannt.

### Treibgashaltige Inhalatlonsaerosols

Die im Rahmen der erfindungsgemäßen Verwendung einsetzbaren treibgashaitigen Inhalativnsaerosola können **I** im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclapropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1,1,1, 2-Tetrafluorethan), TG227 (1,1,1,2,3,3, 3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solvontien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

### Treibgasfreie Inhalationslösungen

Die erfindurigsgemäße Verwendung von Verbindungen der Formel **I** erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Cosolventien und/oder weitere Hilfsstoffe zugesetzt werden. Bevorzugte Cosolventien sind solche, die Hydroxylgruppen oder andere polare Gruppen enthalten, beispielsweise Alkohole - insbesondere lsapropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin. Ölsäure, Sorbitanester, wie Polysorbate, Polyvinylpyrrolidon sonstige Stabilisatoren; Komplexbildner, Antioxidantien und/oder Konservierungsstoffe, die die Verwendungsdauer der fertigen Arznejmittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet. Vitamin A, Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid. Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegsericrankung, COPD oder Asthma enthalten, ein Pteridin und ein oder mehrere Kombinationspartner ausgewählt aus der oben beschriebenen Gruppe, bereit gestellt.

### EXPERIMENTELLER TEIL

Für die hergestellte Verbindungen liegen in der Regel IR-, ¹H-NMR und/oder Massenspektren vor. Wenn nicht anders angegeben, werden R_{f}-Werte unter Verwendung von DGFertigplatten Kieselgel 60 F254 (E. Merck, Darmstadt. Artikel-Nr, 1,05714) ohne Kammersättigung bestimmt.
Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wird Kieselgel der Firma Millipore (MATREX^{™}, 35-70 µm) verwendet.
Die angegebenen HPLC-Daten werden unter nachstehend angeführten Parametern gemessen:

**Methode A:**

| Zeit (min) | Volumenprozent Wasser (mit 0.1% Ameisensäure) | Volumenprozent Acetonitril (mit 0.1% Ameisensäure) |
|---|---|---|
| 0 | 95 | 5 |
| 9 | 10 | 90 |
| 10 | 10 | 90 |
| 11 | 95 | 5 |

Analytische Säule: Zorbax-Säule (Agilent Technologies). SB (Stable Bond) C18: 3.5 µm; 4.6 x 75 mm; Säulentemperatur: 30°C; Fluss: 0.8 mL / min; Injektionsvolumen: 5 µL; Detektion bei 254 nm

**Methode B:**

| Zeit (min) | Volumenprozent Wasser (mit 0.1 % Ameisensäure) | Volumenprozent Acetonitril (mit 0.1 % Ameisensäure) |
|---|---|---|
| 0 | 95 | 5 |
| 4.5 | 10 | 90 |
| 5 | 10 | 90 |
| 5.5 | 90 | 10 |

Analytische Säule: Zorbax-Säule (Agilent Technologies), SB (Stable Bond) C18; 3.5 µm; 4.6 x 75 mm; Säulentemperatur. 30°C; Fluss: 1.6 mL / min; Injektionsvolumen: 5 µL: Detektion bei 254 nm

**Methode C.**

| Zeit (min) | Volumenprozent Wasser (mit 0.1% Ameisensäure) | Volumenprozent Acetonitril (mit 0.1% Ameisensäure) |
|---|---|---|
| 0 | 95 | 5 |
| 4.5 | 10 | 90 |
| 5 | 10 | 90 |
| 5,5 | 90 | 10 |

Analytische Säule: Zorbax-Säule (Agilent Technologies), SB (Stable Bond) C18; 3.5 µm; 4.6 x 75 mm; Säulentemperatur: 30°C; Fluss: 1.6 mL / min; Injektionsvolumen: 5 µL; Detektion bei 254 nm

**Methode D:**

| Zeit (min) | Volumenprozent Wasser (mit 0.04% TFA) | Volumenprozent Acetonitril (mit 0.04% TFA) |
|---|---|---|
| 0 | 80 | 20 |
| 15 | 20 | 80 |
| 17 | 20 | 80 |

Analytische Säule: Symmetry C8 Waters - 4.6 X 150 mm; 5 micron, Fluss: 1.3 ml/min, Säulentemperatur: 25°C, Detektion bei 254 nm.

Bei präparativen HPLC-Reinigungen werden in der Regel die gleichen Gradienten verwendet, die bei der Erhebung der analytischen HPLC-Daten benutzt wurden.
Die Sammlung der Produkte erfolgt massengesteuert, die Produkt enthaltenden Fraktionen werden vereinigt und gefriergetrocknet.
Falls nähere Angaben zur Konfiguration fehlen, bleibt offen, ob es sich um reine Enantiomere handelt oder ob partielle oder gar völlige Racemisierung eingetreten ist,

In den Versuchsbeschreibungen werden die folgenden Abkürzungen verwendet:
- Cyc: Cyclohexan
- DCM: Dichlormethan
- DIPE: Diisopropylether
- DMF: *N,N*-Dimethylformamid
- EtOAc: Essigsäureöthylester
- EtOH: Ethanol
- AcOH: Essigsäure
- i.vac.: in vacuo (im Vakuum)
- MeOH: Methanol
- MTBE: *tert*-Butylmethylether
- NaOAc: Natriumacetat
- RT: Raumtemperatur
- TBTU: O-(Benzotriazol-1-yl)-*N,N,N*'*,N*'-tetramethyluronium-tetrafluoraborat
- THF: Tetrahydrofuran

### Baustein A1

### 4-(2-Oxo-1,2,4,5-tetrahydro-1,3-benzdiazepin-3-yl)-pipeddin-1-carbonsäure-(R)-1-carboxy-2-(3-ethyl-4-hydroxy-5-methyl-phenyl)-ethylester

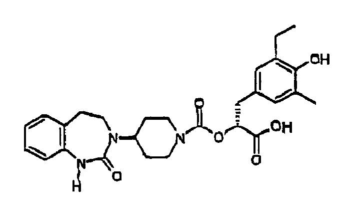

### A1a) 2-Ethyl-6-methyl-phenol

Zu einer Lösung von 30 g (222 mmol) 2-Ethyl-6-methyl-anilin in 135 mL EtOH wurden bei 0°C 19.4 mL (0.23 mmol) konzentrierte HCl und eine Lösung von 16.1 g (0.23 mmol) Natriumnitrit in Wasser (ca. 70 mL) zugegeben und 15 min gerührt. Dieses Gemisch wurde bei 45°C zu einer Lösung 10.5 mL konzentrierter H₂SO₄ in 300 mL Wasser zugegeben und am Ende der Zugabe auf 70°C erhitzt. Die wässrige Phase wurde auf RT abgekühlt und mit EtOAc erschöpfend extrahiert. Die kombinierten organischen Phasen wurden mit 1 M NaOH-Lösung extrahiert. Die wässrige Phase wurde mit DCM gewaschen, mit 4 N HCl-Lösung auf pH 1 angesäuert und mit DCM extrahiert, Die organische Phase wurde mit gesättigter NaCl-Lösung gewaschen, über Na₂SO₄ getrocknet und i.vac. eingeengt. Das Rohprodukt wurde ohne weitere Reinigung in den nachfolgenden Reaktionsschritt eingesetzt.

| | |
|---|---|
| Ausbeute: | 12.0 g (40% d. Theorie) |

### A1b) 4-Brom-2-ethyl-6-methyl-phenol

Zu einer Lösung von 33.6 g (247 mmol) 2-Ethyl-6-methyl-phenol in 350 mL Chloroform wurde bei RT eine Lösung von 12.7 mL (247 mmol) Brom in 10 mL Chloroform zugetropft und das Gemisch 2 h gerührt. Das Reaktionsgemisch wurde mit einer wässrigen NaHSO₃-Lösung versetzt und 20 min gerührt. Die Phasen wurden getrennt und die organische Phase mit gesättigter NaCl-Lösung gewaschen, über Na₂SO₄ getrocknet und i.vac. eingeengt. Säulenchromatographie (Kieselgel, Cyc/EtOAc 9:1) ergab das Produkt.

| | |
|---|---|
| Ausbeute: | 39.8 g (75% d. Theorie) |
| ESI-MS: | (M+H)⁺ = 214/216 (Br) |
| Retentionszeit (HPLC-MS): | 6.3 min (Methode D) |

### A1c) 2-Benzyloxy-5-brom-1-ethyl-3-methyl-benzol

Eine Suspension von 39.8 g (185 mmol) 4-Brom-2-ethyl-6-methyl-phenol, 63.9 g (0.46 mmol) K₂CO₃ und 22.0 mL (185 mmol) Benzylbromid in 450 mL Acetonitril wurde 3 h unter Rückfluss erhitzt, auf RT abgekühlt und i.vac. eingeengt. Der Rückstand wurde mit EtOAc versetzt, die organische Phase mit Wasser und gesättigter NaCl-Lösung gewaschen, über Na₂SO₄ getrocknet und i.vac. eingeengt.

| | |
|---|---|
| Ausbeute: | 54.5 g (96% d. Theorie) |
| ESI-MS: | (M+H)⁺ = 304/306 (Br) |
| Retentionszeit (HPLC-MS); | 9.4 min (Methode D) |

### A1d) (Z,E)-2-Acetylamino-3-(4-benzyloxy-3-ethyl-5-methyl-Phenyl)-acrylsäuremethylester

Hergestellt analog Beispiel 1b aus 50.4 g (165.1 mmol) 2-Benzyloxy-5-bram-l-ethyl-3-methyl-benzol und 28.9 g (198.2 mmol) 2-Acetylamino-acrylsäuremethylester.

| | |
|---|---|
| Ausbeute: | 41,0 g (68% d. Theorie) |
| ESI-MS: | (M+H)⁺ = 368 |
| Retentionszeit (HPLC-MS): | 4.5 min (Methode C) |

### A1e) 3-(4-Benzyloxy-3-ethyl-5-methyl-phenyl)-2-oxo-propionsäure

Zu einer Lösung von 41,0 g (111.6 mmol) (Z,E)-2-Acotylamino-3-(4-benzyloxy-3-ethyl-5-methyl-phenyl)-acrylsäuremethyloster in 300 mL 1,4-Dioxan wurden 200 mL 4 M HCl gegeben und die Reaktionslösung 7 h auf 130°C (Badtemperatur) erhitzt. Die organische Phase wurde heiß abgetrennt, i.vac. eingeengt und der erhaltene Rückstand aus Toluol umkristallisiert.

| | |
|---|---|
| Ausbeute: | 9.6 g (28% d. Theorie) |
| ESI-MS: | (M+H)⁺ = 312 |
| Retentionszeit (HPLC-MS): | 4.1 min (Methode C) |

### A1f) (R)-3-(4-Benzyloxy-3-ethyl-5-methyl-phenyl)-2-hydroxy-propionsäure

Unter einer Argonatmosphäre wurde eine Lösung von 9.59 g (30.7 mmol) 3-(4-Benzyloxy-3-ethyl-5-methyl-phenyl)-2-oxo-propionsäure in 25 mL THF mit 4.26 mL (31.0 mmol) Triethylamin versetzt. 5 min nachgerührt und auf -30°C (interne Temperatur) abgekühlt, Eine Lösung von 19.7 g (61.0 mmol) (1*R*)-*B*-Chlordiisopinocampheylboran in 35 mL wurde zugetropft und die Reaktionslösung nach beendeter Zugabe 30 min ohne Kühlung nachgerührt. Man versetzte mit 15 mL 4 N NaOH (Temperaturanstieg auf 20°C), 5 min nachgerührt, auf 0°C abgekühlt, mit 50 mL MTBE versetzt und 20 min nachgerührt. Die organische Phase wurde abgetrennt und über Na₂SO₄ getrocknet. Nach Entfernen des Trocken- und Lösungsmittels wurde der Rückstand ohne Reinigung weiter umgesetzt.

| | |
|---|---|
| Ausbeute: | 10.3 g (100% d. Theorie) |
| ESI-MS: | (M-H)⁻ = 313 |
| Retentionszeit (HPLC-MS): | 4.2 min (Methode C) |

### A1g) (R)-3-(4-Benzyloxy-3-ethyl-5-methyl-phenyl)-2-hydroxy-propionsäuremethylester

Hergestellt analog Beispiel 1e aus 10.3 g (30.7 mmol) (R)-3-(4-Benzyloxy-3-ethyl-5-methyl-phenyl)-2-hydroxy-propivnsäure und 4.71 mL (64.5 mmol) Thionylchlorid. Das erhaltene Rohprodukt wurde ohne Reinigung weiter umgesetzt.

### A1h) 4-(2-Oxo-1,2,4,5-tetrahydro-1,3-benzdiazepin-3-yl)-piperidin-1-carbonsäure-(R)-2-(4-benzyloxy-3-ethyl-5-methyl-phenyl)-1-methoxycarbonyl-ethylester

Zu einer auf 60°C (Badtemperatur) erwärmten Lösung von 75 mL Pyridin wurden innerhalb 10 min eine Lösung von 7.12 g (34.3 mmol) 4-Nitrophenyl-chloroformat in 30 mL THF zugegeben, 10 min nachgerührt und dann eine Lösung von 10.0 g des Rohproduktes aus Beispiel A1 g in 50 mL Pyridin zugetropft. Man rührte 1 h nach, versetzte mit 6.72 g (27,4 mmol) 3-Piperidin-4-yl-1,3,4,5-tetrahydro-1,3-benzdiazepin-2-on und erhöhte die Badtemperatur auf 100°C (2 h). Der entstandene Niederschlag wurde filtriert, das Filtrat i.vac. eingeengt, der Rückstand mit 150 mL EtOAc versetzt, die organische Phase zweimal mit je 50 mL 1 M KHSO₄-Lösung und zehnmal mit je 50 mL 15% K₂CO₃-Lösung gewaschen und über Na₂SO₄ getrocknet. Nach Entfernen des Trocken- und Lösungsmittels wurde der Rückstand chromatographisch (Kieselgel, EtOAc/Cyc 2:1) gereinigt.

| | |
|---|---|
| Ausbeute: | 2.28 g (14% d. Theorie) |
| ESI-MS: | (M+H)⁺ = 600 |
| Retentionszeit (HPLC-MS): | 5,4 min (Methode C) |

### A1i) 4-(2-Oxo-1,2,4,5-tetrahydro-1,3-benzdiazepin-3-yl)-piperidin-1-carbonsäure-(R)-2-(4-benzyloxy-3-ethyl-5-methyl-phenyl)-1-carboxy-ethylester

Zu einer Lösung von 800 mg (1.33 mmol) 4-(2-Oxo-1,2,4,5-tetrahydro-1,3-benzdiazepin-3-yl)-piperidin-1-carbonsäure-(R)-2-(4-benzyloxy-3-ethyl-5-methyl-phenyl)-1-methoxycarbonyl-ethylester in 15 mL THF wurde eine Lösung von 50 mg (2.09 mmol) LiOH in 5 mL Wasser gegeben und das Reaktionsgemisch 1 h bei RT gerührt. Man engte i.vac ein, nahm den Rückstand in 50 mL Wasser auf und versetzte mit 2 M HCl bis zur sauren Reaktion. Der ausgefallene Niederschlag wurde abfiltriert, mit Wasser gewaschen und getrocknet. Weitere Reinigung erfolgte durch Auskochen mit 150 mL Wasser, Filtration und erneutem Trocknen.

| | |
|---|---|
| Ausbeute; | quantitativ |
| ESI-MS: | (M+H)⁺ = 586 |
| Retentionszeit (HPLC-MS): | 4.8 min (Methode C) |

### A1k) 4-(2-Oxo-1,2,4,5-tetrahydro-1,3-benzdiazepin-3-yl)-piperidin-1-carbonsäure-(R)-1-carboxy-2-(3-ethyl-4-hydroxy-5-methyl-phenyl)-ethylester

810 mg (1.38 mmol) 4-(2-Oxo-1,2,4,5-tetrahydro-1,3-benzdiazepin-3-yl)-piperidin-1-carbonsäure-(R)-2-(4-benzyloxy-3-ethyl-5-mothyl-phenyl)-1-carboxy-ethyloster in 25 mL MeOH wurden mit 80 mg 10% Pd/C versetzt und bei RT und 3 bar Wasserstoff bis zum Reaktionsstillstand hydriert, Der Katalysator wurde abgesaugt und das Lösungsmittel i.vac. eingeengt. Der Rückstand wurde mit DIPE verrieben, abgesaugt und getrocknet.

| | |
|---|---|
| Ausbeute: | 639 mg (93% der Theorie) |
| ESI-MS: | (M+H)⁺ = 496. |
| Retentionszeit (HPLC-MS): | 3.7 min (Methode C) |

### Baustein A2

### 4-(2-Oxo-1,2,4,5-tetrahydro-1,3-benzdiazepin-3-yl)-piperidin-1-carbonsäure-(R)-1-carboxy-2-(4-hydroxy-3-methoxy-5-methyl-phenyl)-ethylester

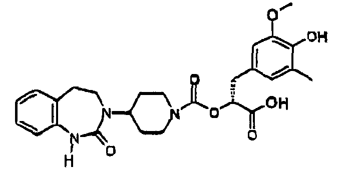

### A2a) 4-Brom-2-methoxy-6-methyl-phenol

Zu einer Lösung von 42.3 g (0.31 mol) 2-Methoxy-6-methyl-phanol in 450 mL AcOH wurde innerhalb von 5.5 h eine Lösung von 56.2 g (0.32 mol) *N*-Bromsuccinimid in 1700 mL AcOH zugetropft und das Gemisch 16 h bei RT gerührt, Das Reaktionsgemisch wurde i.vac. eingeengt und der Rückstand in DCM aufgenommen. Die organische Phase wurde mit 5% NaHCO₃- und gesättigter NaCl-Lösung gewaschen, über Na₂SO₄ getrocknet und i.vac. eingeengt. Das rote Öl wurde ohne weitere Reinigung in den nachfolgenden Reaktionsschritt eingesetzt.

| | |
|---|---|
| Ausbeute; | 65.9 g (66% d. Theorie) |
| R_{f} = | 0.32 (Kieselgel, Hexan/EtOAc 4:1) |
| Retentionszeit (HPLC-MS); | 11.1 min (Methode D) |

### A2b) 2-Benzyloxy-5-brorn-1-methoxy-3-methyl-benzol

Zu einer Lösung von 65.9 g (0.26 mol) 4-Brom-2-methoxy-6-methyl-phenol in 330 mL DMF wurden bei RT 45.7 g (0.33 mol) K₂CO₃ und eine Lösung von 40.3 mL (0.33 mol) Benzylbromid zugegeben und das Gemisch 18 h bei RT gerührt. Das Gemisch wurde filtriert, i.vac. eingeengt und der Rückstand in Diethylether aufgenommen. Die organische Phase wurde mit Wasser, 5% Na₂CO₃- und NaCl-Lösung gewaschen, über Na₂SO₄ getrocknet und i.vac. eingeengt. Das Rohprodukt wurde ohne weitere Reinigung in den nachfolgenden Reaktionsschritt eingesetzt.

| | |
|---|---|
| Ausbeute: | 92.2 g (81 % d. Theorie) |
| R_{f} = | 0,56 (Kieselgel, Hexan/EtOAc 4:1) |
| Retentionszeit (HPLC-MS): | 16.3 min (Methode D) |

### A2c) 4-Senzyloxy-3-methoxy-5-methyl-benzaldehyd

Zu einer Lösung von 61.2 g (119.5 mmol) 2-Benzyloxy-5-brom-1-methoxy-3-methylbenzol in 240 mL THF wurden bei -75°C 96 mL (240 mmol) n-Butyllithium (2.5 M in Hexan) zugetropft und das Gemisch 15 min bei -75 °C gerührt. Eine Lösung von 31 mL (402 mmol) DMF in 30 mL THF wurde zugetropft, das Gemisch auf 0°C erwärmt und weitere 2 h gerührt. Die Reaktion wurde mit gesättigter NH₄Cl-Lösung versetzt, mit 150 mL Wasser verdünnt und die Phasen getrennt. Die wässrige Phase wurde erschöpfend mit Diethylether extrahiert. Die vereinigten organischen Phasen wurden mit gesättigter NaCl-Lösung gewaschen, über Na₂SO₄ getrocknet und i.vac. eingeengt. Säutenchromatographie (Kieselgel, Hexan/EtOAc 85:15) ergab das Produkt als gelbes Öl.

| | |
|---|---|
| Ausbeute: | 27.1 g (88% d. Theorie) |
| R_{f} = | 0.32 (Kieselgel, Hexan/EtOAc 4:1) |
| Retentionszeit (HPLC-MS): | 13.3 min (Methode D) |

### A2d) 2-Acetylamino-3-(4-benzyloxy-3-methoxy-5-methyl-phenyl)-acrylsäure

Eine Suspension von 27.0 g (105.4 mmol) 4-Benzyloxy-3-methoxy-5-methyl-benzaldehyd, 18.5 g (158.0 mmol) N-Acetylglycin und 12.96 g (158.0 mmol) NaOAc in 120 mL Essigsäureanhydrid wurde unter Stickstoff 3.5 h auf 115°C erwärmt. Bei 100 °C wurden langsam 60 mL Wasser zugetropft und das Gemisch 1 h gerührt. Das Reaktionsgemisch wurde auf RT gekühlt, in Wasser gegossen und die wässrige Phase mit EtOAc erschöpfend extrahiert. Die vereinigten organischen Phasen wurden mit gesättigter NaCl-Lösung gewaschen, über Na₂SO₄ getrocknet und i.vac. eingeengt. Der Rückstand wurde mit Isopropanol verrieben, der erhaltene Feststoff mit Isopropanol, Diethylether und wenig Aceton gewaschen und i.vac. bei 45°C getrocknet.

| | |
|---|---|
| Ausbeute: | 21.2 (57% d. Theorie) |
| R_{f} = | 0.24 (Kieselgel, Hexan/EtOAc 4:1) |
| Retentionszeit (HPLC-MS): | 9.4 min (Methode D) |

### A2e) 3-(4-Benzyloxy-3-methoxy-5-methyl-phenyl)-2-oxo-propionsäure

Das Produkt wurde analog zu Beispiel 1c ausgehend von 20.0 g (56,3 mmol) 2-Acotylamino3-(4-benzyloxy-3-methoxy-5-methyl-phenyl)-acrylsäure erhalten. Das Rohprodukt wurde ohne weitere Reinigung in den nachfolgenden Reaktionsschritt eingesetzt.

| | |
|---|---|
| Ausbeute: | 15.6 g (53% d. Theorie) |
| Retentionszeit (HPLC-MS): | 11.9 min (Methode D) |

### A2f) (R)-3-(4-Benzyloxy-3-methoxy-5-methyl-phenyl)-2-hydroxy-propionsäure

Das Produkt wurde analog zu Beispiel 1d ausgehend von 16,0 g (50.90 mmol) 3-(4-Benzyloxy-3-methoxy-5-methyl-phenyl)-2-oxo-propionsäure hergestellt.

| | |
|---|---|
| Ausbeute: | 7.63 g (47% d. Theorie) |
| Retentionszeit (HPLC-MS): | 9.8 min (Methode D) |

### A2g) (R)-3-(4-Benzyloxy-3-methoxy-5-methyl-phenyl)-2-hydroxy-propionsäure-methylester

Das Produkt wurde analog zu Beispiel 1e ausgehend von 7.6 g (24.02 mmol) (R)-3-(4-Benzyloxy-3-methoxy-5-methyl-phenyl)-2-hydroxy-propionsäure hergestellt.

| | |
|---|---|
| Ausbeute: | 6,84 g (86% d. Theorie) |
| Retentionszeit (HPLC-MS): | 11.7.min (Methode D) |

### A2h) 4-(2-Oxo-1,2,4,5-tetrahydro-1,3-benzdiazepin-3-yl)-piperidin-1-carbonsäure-(R)-2-(4-benzyloxy-3-methoxy-5-methoxy-5-methyl-phenyl)-1-methoxycarbonyl-ethylester

Das Produkt wurde analog zu Beispiel 1f ausgehend von 6.8 g (20.6 mmol) (R)-3-(4-Benzyloxy-3-methoxy-5-methyl-phonyl)-2-hydroxy-propionsäure-mathyl-ester in Acetonitril hergestellt.

| | |
|---|---|
| Ausbeute: | 8.16 g (66% d. Theorie) |
| ESI-MS: | (M+H)⁺ = 602 |
| Retentionszeit (HPLC-MS): | 14.1 min (Methode D) |

### A2i) 4-(2-Oxo-1,2,4,5-tetrahydro-1,3-benzdiazepin-3-yl)-piperidin-1-carbonsäure-(R)-2-(4-benzyloxy-3-methoxy-5-methyl-phenyl)-1-carboxy-ethylester

Das Produkt wurde analog zu Beispiel 1g ausgehend von 8.16 g (13.65 mmol) 4-(2-Oxo-1,2,4,5-tetrahydro-1,3-benzdiazepin-3-yl)-piperidin-1-carbonsäure-(R)-2-(4-berleoxy-3-methoxy-5-methyl-phenyl)-1-methoxycarbonyl-ethylester hergestellt.

| | |
|---|---|
| Ausbeute: | 7.83 g (98% d. Theorie) |
| ESI-MS: | (M+H)⁺ = 588 |
| Retentionszeit (HPLC-MS): | 12.2 min (Methode D) |

### A2k) 4-(2-Oxo-1,2,4,5-tetrahydro-1,3-benzdiazepin-3-yl)-piperidin-1-carbonsäure-(R)-1-carboxy-2-(4-hydroxy-3-methoxy-5-methyl-phenyl)-ethylester

Das Produkt wurde analog zu Beispiel 1h ausgehend von 7.80 g (13.27 mmol) 4-(2-Oxo-1,2,4,5-tetrahydro-1,3-benzdiazepin-3-yl)-piperidin-1-carbonsäure-(R)-2-(4-benzyloxy-3-methoxy-5-metnyl-phenyl)-1-carboxy-ethytester hergestellt.

| | |
|---|---|
| Ausbeute: | 5.33 g (80% d. Theorie) |
| ESI-MS: | (M+H)⁺ = 498 |
| Retentionszeit (HPLC-MS); | 8.4 min (Methode D) |

### Beispiel 1

### 4-(2-Oxo-1,2,4,5-tetrahydro-1,3-benzdiazepin-3-yl)-piperidin-1-carbonsäure-(R)-2-[4-(4-ethoxycarbonyl-phenyl)-piparazin-1-yl]-1-(4-hydroxy-3,5-dimethyl-benzyl)-2-oxo-ethylester

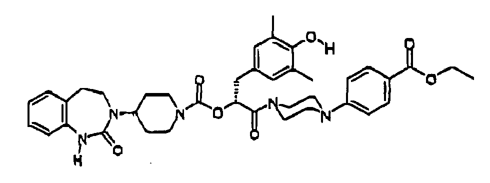

### 1a) 2-Benzyloxy-5-brom-1,3-dimethylbenzol

Zu einer Lösung von 50.0 g (249 mmol) 2,6-Dimethyl-4-bromphenol in 500 mL DMF wurden 39.9 g (286 mmol) K₂CO₃ gegeben und 20 min nachgerührt. Dann wurden 34.0 mL (286 mmol) Benzylchlorid langsam zugetropft und das Reaktionsgemisch 3 h bei 100°C Badtemperatur gerührt. Nach beendeter Umsetzung wurde auf 500 mL Wasser gegossen und mit EtOAc erschöpfend extrahiert. Die organischen Phasen wurden vereint, über Na₂SO₄ getrocknet und i.vac. eingeengt.

| | |
|---|---|
| Ausbeute: | quantitativ |
| GC-MS; | (M⁺) = 290/292 (Br) |
| R_{f}= | 0.87 (Kieselgel, Cyc/EtOAc 3:1) |

### 1b) 2-Acatylamino-3-(4-benzyloxy-3,5-dimethyl-Dhenyl)-acrylsäuremethylester

Unter SfickstoffiÜnosphäre wurde eine Mischung aus 40.0 g (137 mmol) 2-Benzyloxy-5-brom-1,3-dimethylbenzol und 24.1 g (165 mmol) 2-Acetylamino-acrytsäuremethylester in 420 mL Triethylamin und 200 mL Acetonitril mit 3.5 g (11,2 mmol) Tri-o-tolyi-phosphan und 2.5 g (11,1 mmol) Pd(OAc)₂ versetzt und 18 h bei 80°C gerührt. Der Niederschlag wurde abgesaugt, das Filtrat i.vac. eingeengt und mit 800 mL DCM und 800 mL Wasser versetzt, Die organische Phase wurde abgetrennt, über Na₂SO₄ abgesaugt, das Lösungsmittel i.vac. entfernt, der Rückstand mit EtOAc verrührt, abgesaugt und i. vac getrocknet.

| | |
|---|---|
| Ausbeute: | 31.1 g (64% der Theorie) |
| ESI-MS: | (M+H)⁺ = 354 |
| Retentionszeit (HPLC-MS): | 8.6 min (Methode A) |

### 1 c) 3-(4-Benzyloxy-3,5-dimethyl-phenyl)-2-oxo-propionsäure

31.1 g (88.1 mmol) 2-Acetylamino-3-(4-benzyloxy-3,5-dimethyl-phenyl)-acrylsäure-methylester in 150 mL 1,4-Dioxan wurden mit 125 mL 4 M HCl versetzt. 7 h unter Rückfluss und über Nacht bei RT gerührt, Der Niederschlag wurde abgesaugt, mit Wasser gewaschen und bei 45°C im Vakuumtrockenschrank getrocknet.

| | |
|---|---|
| Ausbeute: | 14.3 g (54 % der Theorie) |
| EI-MS: | (M)⁺ = 298 |
| Retentionszeit (HPLC-MS): | 9.0 min (Methode A) |

### 1d) (R)-3-(4-Benzoyl-3,5-dimethyl-phenyl)-2-hydroxy-propionsäure

Unter einer Stickstoffatmosphäre wurde eine Lösung von 14.3 g (47,8 mmol) 3-(4-Benzyloxy-3,5-dimethyl-phenyl)-2-oxo-propionsäure und 8.3 mL (59.8 mmol) Triethylamin in 170 mL THF bei -35°C mit einer Lösung von 22.1 (69,0 mmol) (1*R*)-8-Chlordiisopinocampheylboran in 70 mL THF innerhalb von 30 min versetzt. Nach beendeter Zugabe wurde das Kältebad entfernt und die Reaktionslösung über Nacht bei RT gerührt. Das Reaktionsgemisch wurde bei 0°C mit 70 mL 1 M NaOH alkalisch gestellt, mit 100 mL MTBE versetzt, 15 min nachgerührt und die Phasen getrennt. Die organische Phase wurde mit 50 mL Wasser und dreimal mit je 50 mL 1 M NaOH gewaschen. Die vereinigten wässrigen Phasen wurden mit halbkonz. HCl sauer gestellt, mit EtOAc erschöpfend extrahiert und die vereinigten organischen Phasen über Na₂SO₄ getrocknet. Nach Entfernen des Trocken- und Lösungsmittels wurde der Rückstand ohne Reinigung weiter umgesetzt.

| | |
|---|---|
| Ausbeute: | 14.0 g (98% der Theorie) |
| ESI-MS: | (M-H)⁻ = 299 |
| Retentionszeit (HPLC-MS); | 7.9 min (Methode A) |

### 1e) (R)-3-(4-Benzyloxy-3,5-dimethyl-phenyl)-2-hydroxy-propionsäuremethylester

Zu einer auf 0°C gekühlten Lösung von 14.0 g (23.3 mmol) (R)-3-(4-Benzoyl-3,5-dimethylphenyl)-2-hydroxy-propionsäure in 150 mL MeOH wurden tropfenweise 2.0 mL (27.4 mmol) SOCl₂ zugegeben und das Reaktionsgemisch 1 h bei RT nachgerührt. Die Reaktionslösung wurde i. vac. eingeengt und der Rückstand chromatographisch (Kieselgel, Cyc/EtOAc 3:1) gereinigt.

| | |
|---|---|
| Ausbeute: | 5.7 g (78% der Theorie) |
| ESI-MS: | (M+NH₄)⁺ = 332 |
| Retentionszeit (HPLC-MS): | 9.1 min (Methode A) |

### 1f) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-methoxycarbonyl-ethylester

Unter Stickstoffatmosphäre wurde zu einer Lösung von 1.17 g (9.58 mmol) 4-Dimethylaminopyridin in 50 mL Pyridin 1,93 g (9.58 mmol) Chlorameisensäure-4-nitrophenylester gegeben, 1.5 h bei RT gerührt, mit 3.0 g (9.58 mmol) (R)-3-(4-Benzyloxy-3,5-dimethylphenyl)-2-hydroxy-propionsäurernethylester versetzt und 20 min bei RT gerührt. Anschließend wurden 2.35 g (9.58 mmol) 3-Piperidin-4-yl-1,3,4,5-tetrahydro-1,3-benzdiazepin-2-on zugegeben und 20 h bei RT gerührt. Das Reaktionsgemisch wurde i. vac. eingeengt, der Rückstand in EtOAc aufgenommen, die organische Phase mit 10% KHSO₄ und gesättigter NaHCO₃-Lösung gewaschen und über Na₂SO₄ getrocknet. Nach Entfernen des Trocken- und Lösungsmittels wurde der Rückstand chromatographisch (Kieselgel, Gradient Cyc/EtOAc 1:1 zu 1:2) gereinigt.

| | |
|---|---|
| Ausbeute: | 3.21 g (57% der Theorie) |
| ESI-MS: | (M+H)⁺ = 586 |
| Retentionszeit (HPLC-MS): | 10.4 min (Methode A) |

### 1g) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester

Eine Lösung von 3.21 g (5.48 mmol) 4-(2-Oxo-1,2,4,5-tetrahydro-1,3-benzdiazepin-3-yl)-pipeddin-1-carbonsäure-(R)-2-(4-benzyloxy-3,5-dimethyl-phenyl)-1-methoxy-carbonyl-ethylester in 80 mL THF wurde mit einer Lösung von 200 mg (8.35 mmol) LiOH in 40 mL Wasser versetzt und 1 h bei RT gerührt. Das Reaktionsgemisch wurde i. vac. eingeengt, der Rückstand in 100 mL Wasser aufgenommen, mit 2 M HCl sauer gestellt, der Niederschlag abgesaugt und im Vakuumtrockenschrank bei 40°C getrocknet.

| | |
|---|---|
| Ausbeute: | quantitativ |
| ESI-MS: | (M+H)' = 572 |
| Retentionszeit (HPLC-MS): | 9.2 min (Methode A) |

### 1 h) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester

3.72 g (6.51 mmol) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester in 50 mL DCM wurden mit 300 mg 10% Pd/C versetzt und bei RT und 3 bar Wasserstoff bis zum Reaktionsstillstand geschüttelt. Der Katalysator wurde abgesaugt und das Lösungsmittel i.vac. eingeengt. Der Rückstand wurde mit DIPE verrieben und abgesaugt.

| | |
|---|---|
| Ausbeute: | 2.41 g (77% der Theorie) |
| ESI-MS: | (M+H)⁺ = 482 |
| Retentionszeit (HPLC-MS): | 7.0 min (Methode A) |

### 1 i) 4-(2-Oxo-1,2,4,5-tetrahydro-1,3-benzdiazepin-3-yl)-piperidin-1-carbonsäure-(R)-2-[4-(4-ethoxycarbonyl-phenyl)-piperazin-1-yl]-1-(4-hydroxy-3,5-dimethyl-benzyl)-2-oxo-ethylester

Eine Lösung von 200 mg (0.42 mmol) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester, 148 mg (0,46 mmol) TBTU und 65 µL (0.46 mmol) Triethylamin in 2 mL DMF wurde bei RT 10 min gerührt. Dann erfolgte die Zugabe von 108 mg (0.46 mmol) 4-Piperazin-1-yl-benzoesäureethylester und die Reaktionslösung wurde 2 h gerührt, Die Reaktionslösung wurde ohne weitere Aufarbeitung via HPLC gereinigt; die das Produkt enthaltenden Fraktionen wurden vereinigt und lyophilisiert.

| | |
|---|---|
| Ausbeute: | 216 mg (75 % der Theorie) |
| ESI-MS: | (M+H)⁺ = 698 |
| Retentionszeit (HPLC-MS): | 4.4 min (Methode B) |

### Beispiel 1.1

### 4-(2-Oxo-1,2,4,5-tetrahydro-1,3-benzdiazepin-3-yl)-piperidin-1-carbonsäure-(R)-2-[4-(4-carboxy-phenyl)-piperazin-1-yl]-1-(4-hydroxy-3,5-dimethyl-benzyl)-2-oxo-ethylester

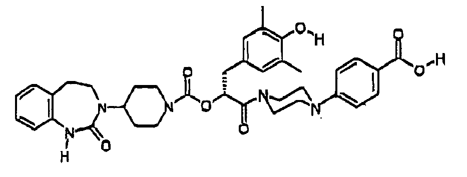

Zu einer Lösung von 50 mg (0,07 mmol) 4-(2-Oxo-1,2,4,5-tetranydro-1,3-benzdiazepin-3-yl)-piperidin-1-carbonsäure-(R)-2-[4-(4-ethoxycarbonyl-phenyl)-piperazin-1-yl]-1-(4-hydroxy-3,5-dimethyl-benzyl)-2-oxo-ethylester in 2 mL THF wurde eine Lösung von 2.6 mg (0.11 mmol) LiOH in 1 mL Wasser gegeben und die Reaktionsmischung über Nacht geschüttelt. Zur Vervollständigung der Reaktion wurden 6.5 µL (0.08 mmol, 35% in Wasser) H₂O₂zugegeben und weitere 4 h bei RT geschüttelt. Man engte i.vac. ein, nahm den Rückstand in Wasser auf und säuerte mit Ameisensäure an. Der entstandene Niederschlag wurde abgesaugt und getrocknet.

| | |
|---|---|
| Ausbeute: | 37 mg (77 % der Theorie) |
| ESI-MS: | (M+H)⁺ = 670 |
| Retentionszeit (HPLC-MS): | 4.0 min (Methode B) |

### Beispiel 2

### 4-(2-Oxo-1,2,4,5-tetrahydro-1,3-benzdiazepin-3-yl)-piperidin-1-carbonsäure-(R)-1-(4-amino-3-chlor-5-trifluormethyhbenzyl)-2-[4-(4-ethoxycarbonyl-phenyl)-piperazin-1-yl]-2-oxo-ethylester

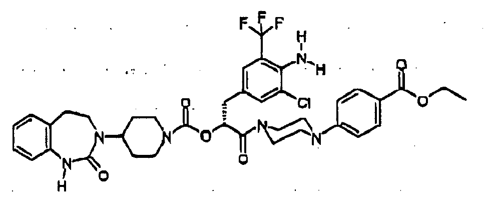

### 2a) (Z,E)-2-Acetylamino-3-(4-amino-3-chlor-5-trifluormethyl-phenyl)-acrylsäure

Zu einer Suspension von 50.0 g (224 mmol) 4-Amino-3-chlor-5-trifluormethyl-benzaldehyd und 27.5 g (335 mmol) NaOAc in 202 mL Acetanhydrid wurden 39.7 g (335 mmol) *N*-Auetylglycin gegeben und das Reaktionsgemisch 1 h auf 115°C erhitzt. Nach Abkühlen auf 80°C wurden 100 mL Wasser zugetropft, wobei die Temperatur des Gemisches bei 80°C gehalten wurde. Die Suspension wurde erneut 40 min auf 95°C erhitzt und dann auf eine Mischung aus 250 mL Toluol und 500 mL Wasser gegeben. Die Suspension wurde bei RT nachgerührt, der Niederschlag abgesaugt und bei 60°C im Umlufttrockenschrank getrocknet.

| | |
|---|---|
| Ausbeute: | 48.8 g (68% der Theorie) |
| ESI-MS: | (M+H)⁺ = 321/323 (Cl) |
| R_{f} = | 0.37 (Kieselgel, DCM/MeOH/AcOH 90:10:1) |

### 2b) 3-(4-Amino-3-chlor-5-trifluormethyl-phenyl)-2-oxo-propionsäure

Eine Suspension von 97.0 g (300 mmol) (*Z,E*)-2-Acetylamino-3-(4-amino-3-chlor-5-trifluormethyl-phenyl)-acrylsäure in 900 mL 1,4-Dioxan und 1050 mL 4 M HCl wurde 8 h auf 100°C erhitzt. Man engte i.vac auf ca. 600 mL ein, kühlte auf RT ab, filtrierte die ausgefallene Substanz ab, wusch diese mit zweimal je 100 mL Wasser und trocknete bei 50°C. Der Rückstand wurde in 850 mL Toluol aufgenommen, unter Rückfluss erhitzt und anschließend im Eisbad gekühlt. Der entstandene Niederschlag wurde filtriert, mit PE gewaschen und im Umlufitrorkenischrank bei 50°C getrocknet.

| | |
|---|---|
| Ausbeute: | 63.0 g (74% der Theorie) |
| ESI-MS: | (M-H)⁻ = 280/282 (Cl) |
| R_{f} = | 0.21 (Kieselgel, DCM/MeOH/NH₃ 80:20:2) |

### 2c) (R)-3-(4-Amino-3-chlor-5-trifluormethyl-phenyl)-2-hydroxy-propionsäure

Zu einer auf ca.-30°C gekühlten Lösung von 63.0 g (224 mmol) 3-(4-Amino-3-chlor-5-trifluormethyl-phenyl)-2-oxa-propionsäure und 31.2 mL (224 mmol) Triethylamin in 300 mL THF wurde eine Lösung von 100.0 g (312 mmol) (1*R*)-B-Chlordiisopinocampheylboran in 150 mL THF zugetropft und das Reaktionsgemisch 1.5 h bei dieser Temperatur gehalten und dann innerhalb einer weiteren Stunde auf RT erwärmt. Zum Reaktionsgemisch wurden mit 80 mL 4 M NaOH gegeben, 5 min nachgerührt, auf 0°C gekühlt, mit 300 mL MTBE versetzt, erneut 20 min bei dieser Temperatur nachgerührt und anschließend die Phasen getrennt. Die organische Phase wurde erschöpfend mit Wasser extrahiert, die vereinigten wässrigen Phasen mit 4 M HCl sauer gestellt, erschöpfend mit MTBE extrahiert und die vereinigten organischen Phasen über Na₂SO₄ getrocknet Die THFIMTBE/NaOH Phase wurde mit 4 M HCl sauer gestellt, die Phasen getrennt und die organische Phase i.vac. eingeengt Beide Rückstände wurden vereinigt und ohne Reinigung weiter umgesetzt.
R_{f} = 0.20 (Kieselgel, DCM/MeOH/NHa 80:20:2)

### 2d) (R)-3-(4-Amino-3-chlor-5-trifluormathyl-phenyl)-2-hydroxy-propionsäuremethylester

Das Rohprodukt aus Beispiel 2c (62 g) wurde in 300 mL MeOH gelöst und zu dieser Lösung langsam 3.65 mL (50 mmol) SOCl₂ zugetropft. Das Reaktionsgemisch wurde weitere 3 h bei RT gerührt, dann i.vac. eingeengt, der Rückstand in DCM aufgenommen und über Kieselgel filtriert. Die Lösung wurde i.vac. eingeengt und der Rückstand chromatographisch (Kieselgel, DCM/MeOH/NH₃ 80:20:2) gereinigt. Die das Produkt enthaltenden Fraktionen wurden vereinigt, i.vac. eingeengt, der Rückstand mit PE versetzt, abgesaugt und getrocknet.

| | |
|---|---|
| Ausbeute: | 43.1 g (65% der Theorie über 2 Stufen) |
| ESI-MS: | (M+H)⁺ = 298/300 (Cl) |
| R_{f} = | 0.86 (Kieselgel. DCM/MeOH/NH₃ 80:20:2) |

### 2e) 4-(2-Oxo-1,2,4,5-tetrahydro-1,3-benzdiazepin-3-yl)-piperidin-1-carbonsäure-(R)-2-(4-amirlo-3-chlor-5-trifluormethyl-Phenyl)-1-methoxycarbonyl-ethylester

Unter Stickstoffatmosphäre wurden bei 60°C (Badtemperatur) zu 100 mL Pyridin innerhalb von 10 min eine Lösung von 13.5 g (65.0 mmol) Chlorameisensäure-4-nitrophenylester in 40 mL THF zudosiert, 10 min nachgerührt, dann eine Lösung von 18.0 g (60.5 mmol) (R)-3-(4-Amino-3-chlor-5-trifluormethyl-phenyl)-2-hydroxy-propionsäure-methylester in 50 ml Pyridin zugetropft und das Reaktionsgemisch 1,5 h bei dieser Temperatur gehalten. Dann erfolgte portionenweise die Zugabe von 15.9 g (65.0 mmol) 3-Piperidin-4-yl-1,3,4,5-tetrahydro-1,3-benzdiazepin-2-on. Die Temperatur des Reaktionsgemisches wurde auf 100°C erhöht, dieses 6 h bei dieser Temperatur gehalten und anschließend über Nacht bei RT nachgerührt. Man engte i.vac. ein, nahm den Rückstand in 200 mL EtOAc auf, wusch die organische Phase zweimal mit je 100 mL 1 M KHSO₄-Lösung, zehnmal mit je 50 mL 15% K₂CO₃-Lösung und trocknete diese über Na₂SO₄. Nach Entfernen des Trocken- und Lösungsmittels wurde der Rückstand ohne Reinigung weiter umgesetzt.

| | |
|---|---|
| Ausbeute: | 33.1 g (96% der Theorie) |
| ESI-MS: | (M+H)⁺ = 569/571 (Cl) |
| R_{f} = | 0.72 (Kieselgel, DCM/Cyc/MeOH/NH₃ 70:15:15:2) |

### 2f) 4-(2-Oxo-1,2,4,5-tetrahydro-1,3-benzdiazepin-3-yl)-piperidin-1-carbonsäure-(R)-2-(4-amino-3-chlor-5-trifluormethyl-phenyl)-1-carboxy-ethylester

Zu einer Lösung von 33.0 g (58.0 mmol) 4-(2-Oxo-1,2,4,5 tetrahydro-1,3-benzdiazepin-3-yl)-piporidin-1-carbonsäure-(*R*)-2-(4-amino-3-chlor-5-trifluormethyl-phenyl)-1-methoxycarbonyl-ethyloster in 200 mL THF wurde eine Lösung von 2.11 g (88.0 mmol) LiOH in 100 mL Wasser zugegeben und die Reaktionslösung 3.5 h bei RT gerührt. THF wurde i.vac. entfernt, der wässrige Rückstand zweimal mit MTBE gewaschen, mit 2 M HCl sauer gestellt, erschöpfend mit DCM extrahiert und die vereinigten organischen Phasen über Na₂SO₄ getrocknet. Nach Entfernen des Trocken- und Lösungsmittels wurde der Rückstand bei 65°C in 80 mL Isopropanol gelöst und über Nacht langsam auf RT abgekühlt. Die Suspension wurde im Eisbad gekühlt, abgesaugt, mit wenig Isopropanol und DIPE gewaschen und getrocknet.

| | |
|---|---|
| Ausbeute: | 26.2 g (81 % der Theorie) |
| ESI-MS: | (M+H)⁺ = 555/557 (Cl) |
| R_{f} = | 0.18 (Kieselgel, DCM/Cyc/MeOH/NH₃ 70:15:15:2) |
| Retentionszeit (HPLC): | 4.0 min (Methode B) |

### 2g) 4-(2-Oxo-1,2,4,5-tetrahydro-1,3-benzdiazepin-3-yl)-piperidin-1-carbonsäure-(R)-1-(4-amino-3-chlor-5-trifluormethyl-benzyl)-2-[4-(4-ethoxycarbonyl-phenyl)-piperazin-1-yl]-2-oxo-ethylester

Eine Lösung von 200 mg (0.36 mmol) 4-(2-Oxo-1,2,4,5-tetrahydro-1,3-benzdiazepin-3-yl)-piperidin-1-carbonsäure-(*R*)-2-(4-amino-3-chlor-5-trifluormethyl-phenyl)-1-carboxy-ethylester, 128 mg (0.40 mmol) TBTU und 56 µL (0.40 mmol) Triethylamin in 2 mL DMF wurde bei RT 10 min gerührt. Dann erfolgte die Zugabe von 94 mg (0,40 mmol) 4-Piperazin-1-yl-benzoesäureethylester und die Reaktionslösung wurde 2 h gerührt. Die Reaktionslösung wurde ohne weitere Aufarbeitung via HPLC gereinigt: die das Produkt enthaltenden Fraktionen wurden vereinigt und lyophilisiert.

| | |
|---|---|
| Ausbeute: | 265 mg (95% der Theorie) |
| ESI-MS: | (M+H)⁺ = 7711773 (Cl) |
| Retentionszeit (HPLC): | 4.8 min (Methode B) |

Die nachfolgenden Beispiele beschreiben die Herstellung pharmazeutischer Anwendungsformen, die als Wirkstoff eine beliebige Verbindung der allgemeinen Formel 1 enthalten:

### Beispiel I

### Kapseln zur Pulverinhalation mit 1 mg Wirkstoff

**Zusammensetzung:**

| 1 Kapsel zur Pulverinhalation enthält: | |
|---|---|
| Wirkstoff | 1.0 mg |
| Milchzucker | 20.0 mg |
| Hartgelatinefcapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

### Inhatationslösung für Respimat^{®} mit 1 mg Wirkstoff

**Zusammensetzung:**

| 1 Hub enthält: | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

### Inhalationslösung für Vernebler mit 1 mg Wirkstoff

**Zusammensetzung:**

| 1 Fläschchen enthält: | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

### Treibgas-Dasieraerosol mit 1 mg Wirkstoff

**Zusammensetzung:**

| 1 Hub enthält: | |
|---|---|
| Wirkstoff | 1.0 mg |
| Lecithin | 0.1 % |
| Treibgas ad | 50,0 µl |

### Herstellungsverfahren:

Der mikronisierte Wirkstoff wird in dem Gemisch aus Lecithin und Treibgas homogen suspendiert, Die Suspension wird in einen Druckbehälter mit Dosierventil abgefüllt,

### Beispiel V

### Nasalspray mit 1 mg Wirkstoff

**Zusammensetzung:**

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

### Injektionslösung mit 5 mg Wirksubstanz pro 5 ml

**Zusammensetzung:**

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen: unter Stickstoff-Begasung in Ampullen abfüllen.

### Beispiel VII

### Injektionslösung mit 100 mg Wirksubstanz pro 20 ml

**Zusammensetzung:**

| | |
|---|---|
| Wirksubstanz | 100 mg |
| Monokaliumdihydrogenphosphat = KH₂PO₄ | 12 mg |
| Dinatriumhydrogenphosphat = Na₂HPO₄*2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen,

### Beispiel VIII

### Lyophilisat mit 10 mg Wirksubstanz

**Zusammensetzung:**

| | |
|---|---|
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

**Lösungsmittel für Lyophilisat:**

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

### Tabletten mit 20 mg Wirksubstanz

**Zusammensetzung:**

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässerigen Lösung von Povidon granulieren; mit Magnesiumstearat mischen: auf einer Tablettenpresse abpressen; Tablettengewicht 200 mg.

### Beispiel X

### Kapseln mit 20 mg Wirksubstanz

**Zusammensetzung:**

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen: Mischung auf einer Kapselfüllmaschine in Hartgelatine-Kapseln Größe 3 abfüllen.

### Beispiel XI

### Zäpfchen mit 50 mg Wirksubstanz

**Zusammensetzung:**

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

### Injektionslösung mit 10 mg Wirksubstanz pro 1 mL

**Zusammensetzung:**

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen: unter Stickstoff-Begasung in Ampullen abfüllen.

## Patentansprüche

1. CGRP-Antagonisten der allgemeinen Formel **I** 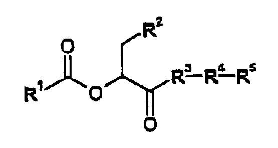 in der
**R¹** eine Gruppe ausgewählt aus 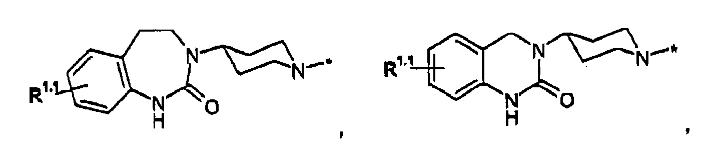 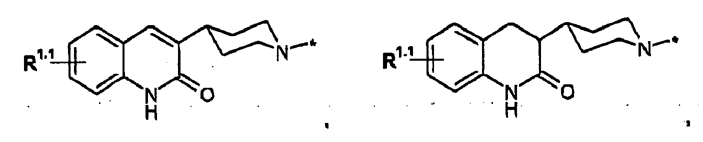 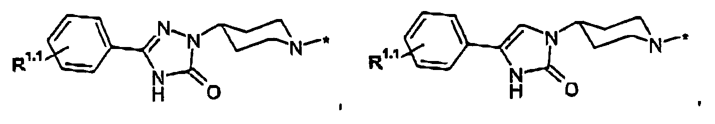 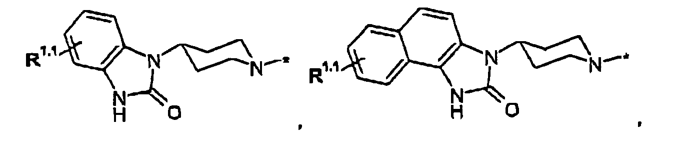 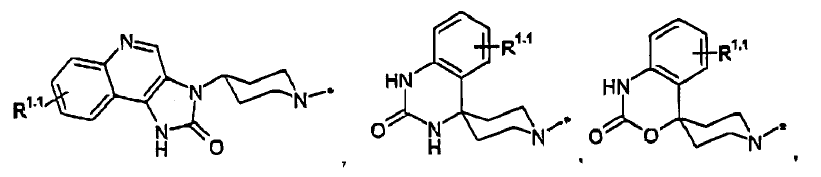 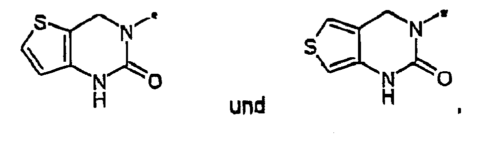 worin
**R^{1.1}** H, Halogen, HO-, F₈C- oder C₁₋₆-Alkyl-O- darstellt,
**R²** eine Gruppe der allgemeinen Formeln **II** 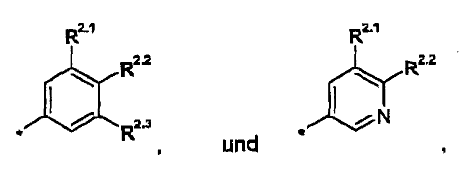 worin
**R^{2.1}** H, Halogen, C₁₋₃-Alkyl-O-, C₁₋₃-Alkyl- oder F₃C-,
**R^{2.2}** H, H₂N-, HO-, H₃C-O-, H-C(O)-O- oder C₁₋₃-Alkyl-C(O)-O-,
**R^{2.3}** H, Halogen, C₁₋₃-Alkyl- oder F₃C- darstellt,
**R³** eine Gruppe der allgemeinen Formeln **III** 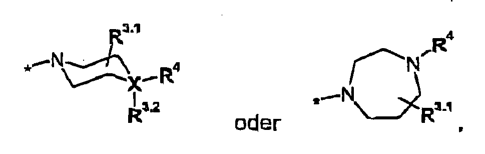 worin
X N oder C,
R^{3.1} H, C₁₋₃-Alkyl- oder R^{3.1.1}-(O)C-.
R^{3.1.1} HO- oder C₁₋₆-Alkyl-O-.
**R^{3.2}** ein freies Elektronenpaar, wenn X = N ist, oder
**R^{3.2}** H oder C₁₋₃-Alkyl- darstellt, wenn X = C ist,
R⁴ eine Gruppe ausgewählt aus 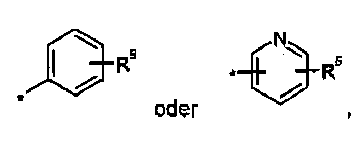
**R⁵**
**R^{5.1} -O-C(O)-,**
**R^{5.1}** H, C₁₋₈-Alkyl-, Phenyl-, Indanyl-, Pyridyl-C₁₋₃-alkylenyl-, HO-C₂₋₄-alkylenyl-, C₁₋₆-Alkyl-O-C₂₋₄-alkylenyl-. HO-C₂₋₄-alkylenyl-O-C₂₋₄-alkylenyl-, C₁₋₈-Alkyl-C₂₋₄-alkylenyl-O-C₂₋₄-alkylenyl-. H₂N-C₂₋₄-alkylenyl-, (C₁₋₆-Alkyl)-NH-C₂₋₄-alkylenyl-, (C₁₋₆-Alkyl)₂N-C₂₋₄-alkylenyl-, H₂N-C(O)-C₁₋₃-alkylenyl-, (C₁₋₆-Alkyl)-NH-C(O)-C₁₋₃-alkylenyl-, (C₁₋₆-Alkyl)₂N-C(O)-C₁₋₃-alkylenyl-, G₁₋₆-Alkyl-C(O)-O-C₁₋₃alkylenyl-, C₁₋₆-Alkyl-O-C(O)-C₁₋₃-alkylenyl-, **R^{5.1.1}**-C(O)-C₁₋₃-alkylenyl- oder **R^{5.1.2}**-C₂₋₄-alkylenyl-.
**R^{5.1.1}** eine Gruppe ausgewählt aus 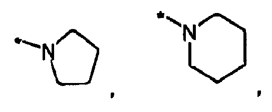
**R^{5.1.2}** eine Gruppe darstellt ausgewählt aus 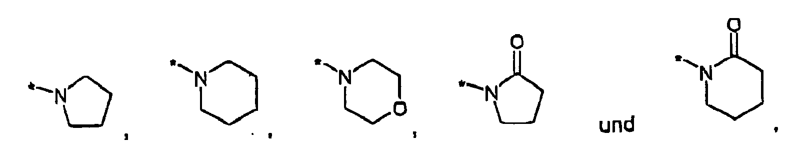
bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. Verbindungen der allgemeinen Formel **I** gemäß Anspruch 1, in der
**R¹** eine Gruppe ausgewählt aus 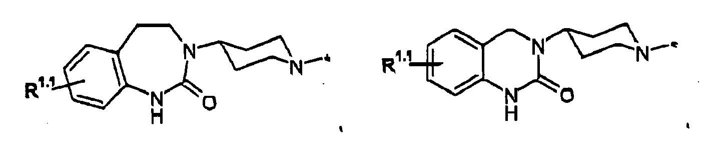 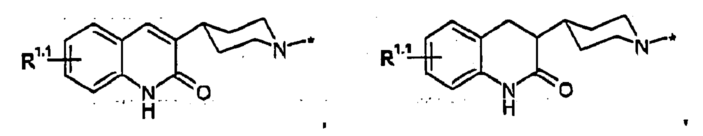 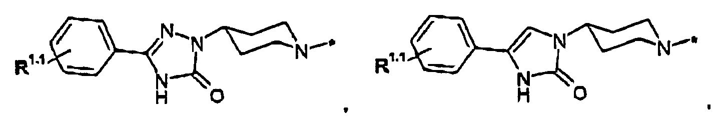 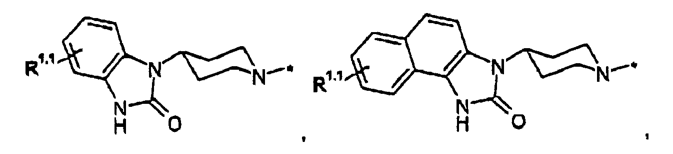 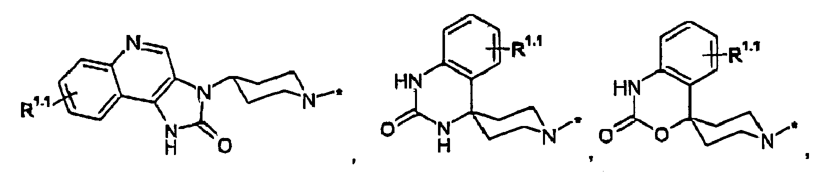 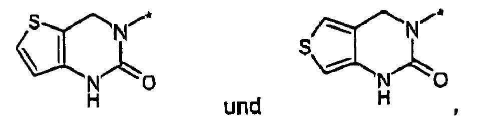 worin
**R^{1.1}** H oder H₃C-O- darstellt,
**R²** eine Gruppe ausgewählt aus 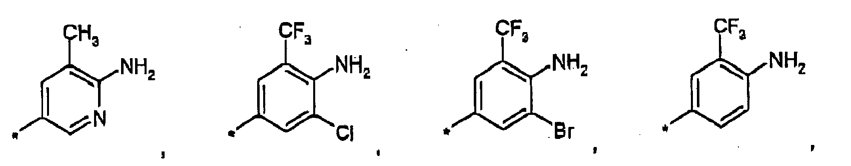 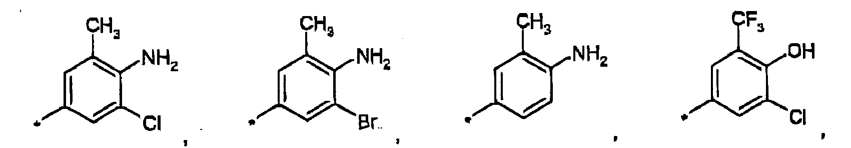 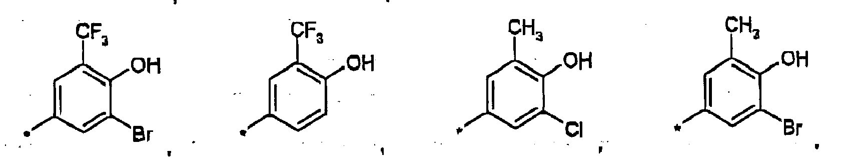 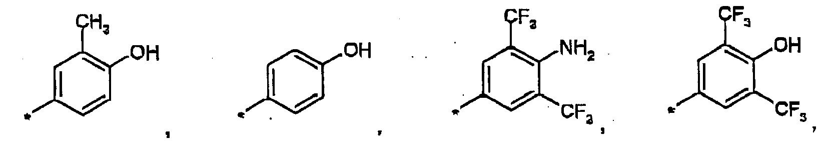 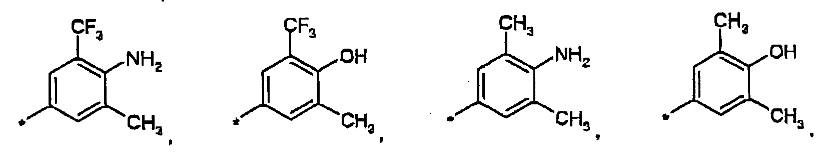 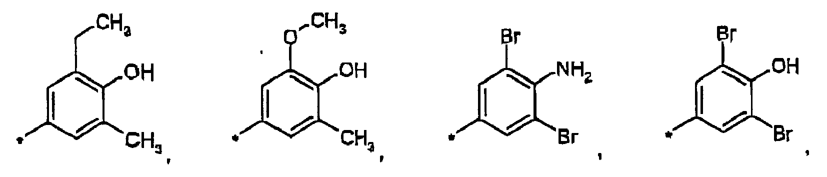 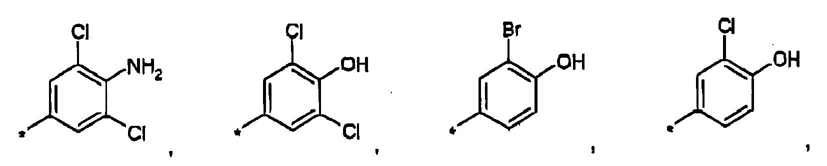 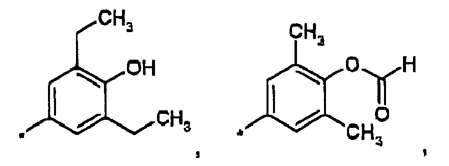 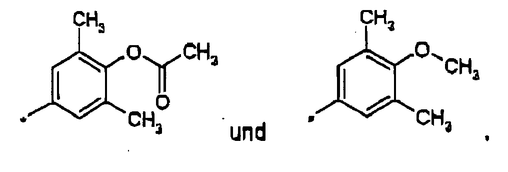
**R³-R⁴** zusammen eine Gruppe ausgewählt aus 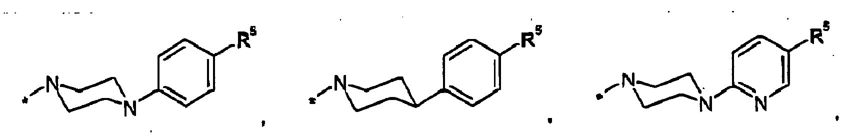 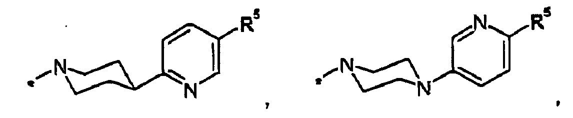 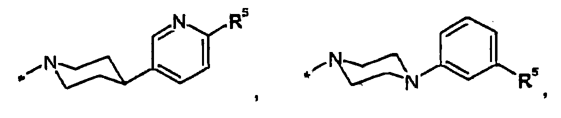 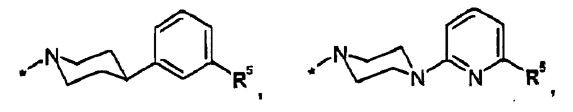 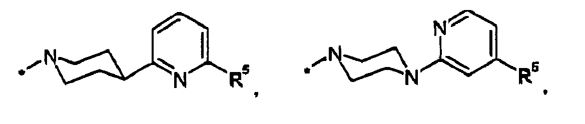 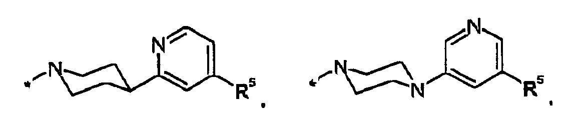 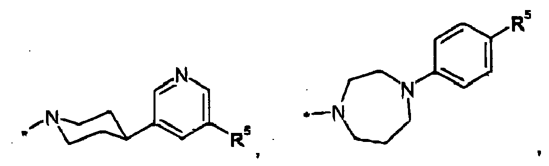 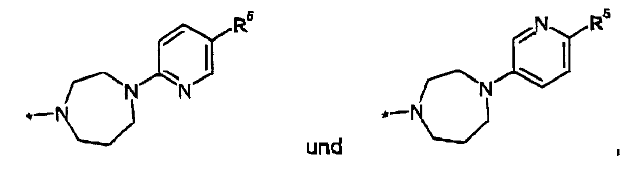
**R⁵**
**R^{5.1}** -O-C(O)- und
**R^{5.1}** H, C₁₋₈-Alkyl-, Phenyl-, Indarlyl-, Pyridyl-C₁₋₃-alkylenyl-, HO-C₂₋₄-alkylenyl-, C₁₋₆-Alkyl-O-C₂₋₄-alkylenyl-, HO-C₂₋₄-alkylenyl-O-C₂₋₄-alkylenyl-, C₁₋₆-Alkyl-C₂₋₄-alkylenyl-O-C₂₋₄-alkylenyl-. H₂N-C₂₋₄-alkylenyl-, (C₁₋₆Alkyl)-NH-C₂₋₄-alkylenyl-. (C₁₋₆-Alkyl)₂N-C₂₋₄-alkylenyl-, H₂N-C(O)-C₁₋₃-alkylenyl-, (C₁₋₆-Alkyl)-NH-C(O)-C₁₋₃-alkylenyl-, (C₁₋₆-Alkyl)₂N-C(O)-C₁₋₃-alkylenyl-, C₁₋₆-Alkyl-C(O)-O-C₁₋₃-alkylenyl-, C₁₋₆-Alkyt-O-C(O)-O-C₁₋₃-alkylenyl-, **R^{5.1.1}**-C(O)-C₁₋₃-alkylenyl- oder **R^{5.1.2}**-C₂₋₄-alkylenyl-,
**R^{5.1.1}** eine Gruppe ausgewählt aus 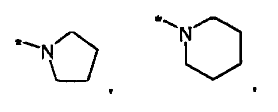
**R^{5.1.2}** eine Gruppe darstellt ausgewählt aus 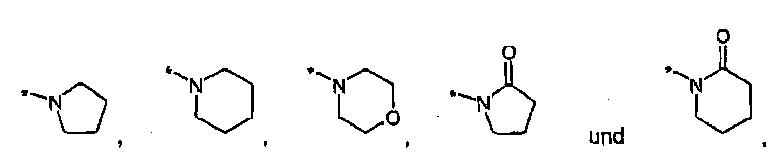
bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

3. Verbindungen der allgemeinen Formel **I** gemäß Anspruch 1, in der
**R¹** eine Gruppe ausgewählt aus 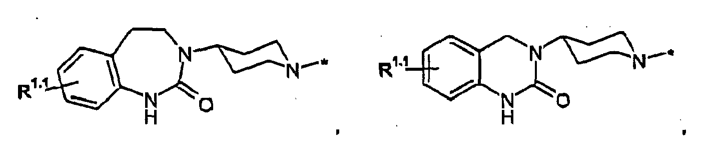 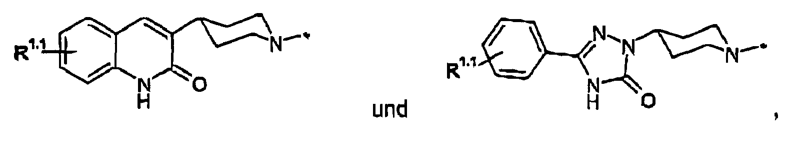
**R²** eine Gruppe ausgewählt aus 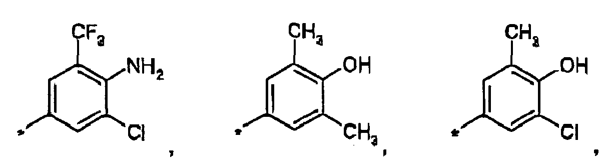 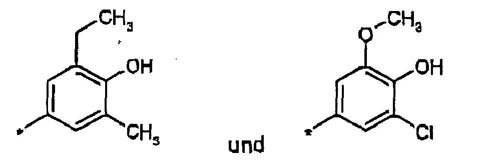
**R³-R⁴** zusammen eine Gruppe ausgewählt aus 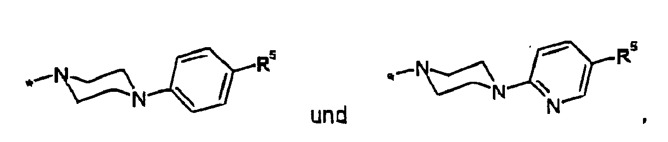
**R^{5.1} -O-C(O)- und**
**R^{5.1}** H, C₁₋₆-Alkyl-, Phenyl-, Indanyl-, Pyridyl-CH₂-, C₁₋₃-Alkyl-O-C₂₋₄-alkylenyl-, C₁₋₃-Alkyl-O-C₂₋₄-alkylenyl-O-C₂₋₄-alkylenyl-, (C₁₋₃-Alkyl)₂N-C₂₋₄-alkylenyl-, (C₁₋₃-Alkyl)₂N-C(O)-C₁₋₃-alkylenyl-, C₁₋₈-Alkyl-C(O)-O-C₁₋₃-alkylenyl-, C₁₋₃-Alkyl-O-C(O)-O-C₁₋₃-alkylenyl-, **R^{5.1.1}**-C(O)-C₁₋₃-alkylenyl- oder **R^{5.1.2}**-C₂₋₄-alkylenyl-,
**R^{5.1.1}** eine Gruppe ausgewählt aus 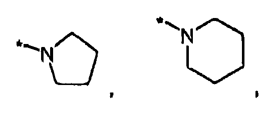
**R^{6.1.2}** eine Gruppe darstellt ausgewählt aus 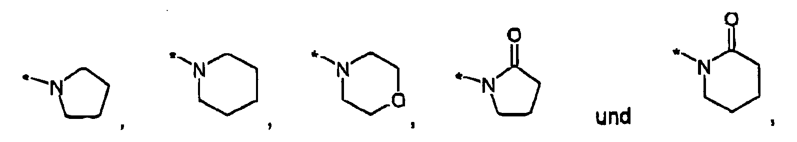
bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

4. Physiologisch verträgliche Salze der Verbindungen nach einem der Ansprüche 1 bis 3 mit anorganischen oder organischen Säuren oder Basen.

5. Arzneimittel, enthaltend eine Verbindung nach mindestens einem der Ansprüche 1 bis 3 oder ein physiologisch verträgliches Salz gemäß Anspruch 4 neben gegebenenfalls einem oder mehreren inerten Trägerstoffen und/oder Verdünnungsmitteln.

6. Verwendung einer Verbindung nach mindestens einem der Ansprüche 1 bis 4 zur Herstellung eines Arzneimittels zur akuten und prophylaktischen Behandlung von Kopfschmerzen, insbesondere Migräne-, Cluster-Kopfschmerz sowie Spannungskopfschmerzen.

7. Verwendung einer Verbindung nach mindestens einem der Ansprüche 1 bis 4 zur Herstellung eines Arzneimittels zur Bekämpfung von nicht-insulinabhängigem Diabetesmellitus (NIDDM).

8. Verwendung einer Verbindung nach mindestens einem der Ansprüche 1 bis 4 zur Herstellung eines Arzneimittels zur Behandlung von cardiovaskulären Erkrankungen, von Morphintoleranz, von Chlostridiumtoxin-bedingten Durchfallerkrankungen, von Erkrankungen der Haut, insbesondere von thermischen und strahjungsbedingten Schäden inklusive Sonnenbrand. Lichen, prurigo, Pruriginöse Toxidermien sowie schwerer Juckreiz, von entzündlichen Erkrankungen, z.B. entzündlichen Gelenkerkrankungen wie Osteoarthritis, rheumatoide Arthritis oder neurogene Arthritis, generalisierten Weichteilrheumatismus (Fibromyalgie), von neurogenen Entzündungen der oralen Mucosa, von entzündlichen Lungenerkrankungen, von allergischer Rhinitis, von Asthma und COPD, von Erkrankungen, die mit einer überschießenden Gefäßerweiterung und **dadurch** bedingter verringerter Gefäßdurchblutung, wie insbesondere Schock oder Sepsis, einhergehen, von chronischen Schmerzerkrankungen wie z.B. diabetische Neuropathien, durch Chemotherapien induzierte Neuropathien, HIV-induzierte Neuropathien, postherpetische Neuropathien, durch Gewebetraumata induzierte Neuropathien, trigeminale Neuralgien, von temporomandibulären Dysfunktionen, von CRPS, RücKenschmerzen, von viszeralen Erkrankungen wie z.B. IBS (irritable bowel Syndrome) oder inflammable bowel syndrome, zur Linderung von Schmerzzuständen im allgemeinen oder zu präventiven oder akut therapeutischen Beeinflussung der durch Gefäßerweiterung und erhöhten Blutfluss verursachten Symptomatik von Hitzewallungen menopausaler, östrogendefzienter Frauen sowie hormonbehandelter Prostatakarzinompatienten und Kastraten.

9. Verfahren zur Herstellung eines Arzneimittels gemäß Anspruch 5, **dadurch gekennzeichnet, dass** auf nichtchemischem Weg eine Verbindung nach mindestens einem der Ansprüche 1 bis 4 in einen oder mehrere inerte Trägerstoffe und/oder Verdünnungsmittel eingearbeitet wird.
